**Europäisches Patentamt**

(19) **European Patent Office**

**Office européen des brevets**

(11) Publication number : **0 387 915 B1**

(12) # EUROPEAN PATENT SPECIFICATION

(45) Date of publication of patent specification :
**10.03.93 Bulletin 93/10**

(21) Application number : **90105190.4**

(22) Date of filing : **18.10.85**

(51) Int. CI.$^5$ : **C07K 15/04,** C12N 15/49,
C07K 1/00, C07K 3/04,
G01N 33/569

(54) **F antigens of the human immunodeficiency virus, and their applications.**

Divisional application 91113062.3 filed on
18/10/85.

(30) Priority : **18.10.84 FR 8416013**
**16.11.84 GB 8429099**
**21.01.85 GB 8501473**

(43) Date of publication of application :
**19.09.90 Bulletin 90/38**

(60) Publication number of the earlier application in
accordance with Art. 76 EPC : **0 201 540**

(45) Publication of the grant of the patent :
**10.03.93 Bulletin 93/10**

(84) Designated Contracting States :
**AT BE CH DE FR GB IT LI LU NL SE**

(56) References cited :
**NATURE, vol. 313, no. 6000, January 1985,
pages 277-284, London, GB; L. RATNER et al.:
"Complete nucleotide sequence of the AIDS
virus, HTLV-III"
NATURE, vol. 313, 7th February 1985, pages
450-458; M.A. MEUSING et al.: "Nucleic acid
structure and expression of the human
AIDS/lymphadenopathy retrovirus"
CELL, vol. 40, January 1985, pages 9-17, MIT;
S. WAIN-HOBSON et al.: "Nucleotide sequ-
ence of the AIDS virus, LAV"**

(73) Proprietor : **INSTITUT PASTEUR**
**25-28, rue du Docteur Roux**
**F-75724 Paris Cédex 15 (FR)**
Proprietor : **CENTRE NATIONAL DE LA
RECHERCHE SCIENTIFIQUE**
**15, quai Anatole France**
**F-75007 Paris (FR)**

(72) Inventor : **Mongagnier, Luc**
**21, rue de Malabry**
**F-92350 LE-PLESSIS-ROBINSON (FR)**
Inventor : **Krust, Bernard**
**7, rue de Madagascar**
**F-75012 PARIS (FR)**
Inventor : **Chamaret, Solange**
**324, rue Lecourbe**
**F-75015 PARIS (FR)**
Inventor : **Clavel, François**
**83, rue de l'Assomption**
**F-75016 PARIS (FR)**
Inventor : **Chermann, Jean-Claude**
**2, Clos d'Ergal**
**F-78310 ELANCOURT (FR)**
Inventor : **Barre-Sinoussi, Françoise**
**50, rue d'Erevan**
**F-92310 ISSY-LES-MOULINEAUX (FR)**
Inventor : **Alizon, Marc**
**71, rue Cardinal Lemoine**
**F-75005 PARIS (FR)**
Inventor : **Sonigo, Pierre**
**23, rue Gutenberg**
**F-75015 PARIS (FR)**
Inventor : **Stewart, Cole**
**4bis, Villa Denise**
**F-92320 CHATILLON (FR)**
Inventor : **Danos, Olivier**
**1, Place Rollet**
**F-75015 PARIS (FR)**
Inventor : **Wain-Hobson, Simon**
**3, rue Jean de la Fontaine**
**F-78180 MONTIGNY LES BRETONNEUX (FR)**

(74) Representative : **Gutmann, Ernest et al**
**S.C. Ernest Gutmann - Yves Plasseraud 67,
boulevard Haussmann**
**F-75008 Paris (FR)**

## Description

The present invention relates to antigens, particularly in a purified form, of the virus of lymphadenopathies (denoted below by the abbreviation LAS) and of the acquired immuno-depressive syndrome (denoted below by the abbreviation AIDS), to a process for producing these antigens, particularly antigens of the envelope of these viruses. The invention also relates to polypeptides, whether glycosylated or not, encoded by said DNA sequences.

The causative agent of LAS or AIDS, a retrovirus, has been identified by F. BARRE-SINOUSSI et al, Science, 220, 868 (1983). It has the following characteristics. It is T-lymphotropic; its preferred target is constituted by Leu 3 cells (or T4 lymphocytes) ; it has reverse transcriptase activity necessitating the presence of Mg + and exhibits strong affinity for poly(adenylate-oligodeoxy-thymidylate)(poly(A)-oligo(dT) 12-18). It has a density of 1.16-1.17 in a sucrose gradient, an average diameter of 139 nanometers; and a nucleus having an average diameter of 41 nanometers. Antigens of said virus, particularly a protein p25 are recognised immunologically by antibodies contained in sera taken up from patients afflicted with LAS or AIDS. The p25 protein, which is a core protein, is not recognised immunologically by antibodies to the p24 protein of the HTLVI and II viruses. The virus is also free of a p19 protein which is immunologically cross-reactive with the p19 proteins of HTLVI and HTLVII.

Retroviruses of this type (sometimes denoted by the generic abbreviation LAV) have been filed in the National Collection of Micro-organism Cultures of the INSTITUT PASTEUR of Paris, under numbers I-232, I-240 and I-241. Virus strains similar to LAV in all respects from the morphological and immunological point of view have been isolated in other laboratories. Reference is made by way of examples to the retrovirus strains named HTLV-III isolated by R.C. GALLO et al., Science, 224, 500 (1984) and by M.G. SARNGADHARAN et al., Science 224, 506 (1984) respectively and to the retrovirus isolated by M. JAY LEVY et al., Science, 225, 840-842 (1984), which virus was designated ARV. For the ease of language the last mentioned viruses, as well as others which have equivalent morphological and immunological properties, will be designated hereafter under the generic designation "LAV". Reference is also made to European patent application filed 14 September 1984, with the priority of British patent application number 83 24800 filed 15 september 1983 as regards a more detailed description of the LAV retroviruses or the like and of the uses to which extracts of these viruses give rise.

Initially the core antigens were the main antigens of the virus lysates or extracts which were recognised by sera of patients infected with AIDS or LAS, in the test systems which had then been used. A p42 protein, presented as consisting of an envelope protein, had been detected too. In the same manner GALLO et al disclosed a p41 protein which was also deemed to be a possible component of the virus envelope.

Processes for obtaining a LAV virus have also been described. Reference may be made particularly to the article already mentioned of F. BARRE-SINOUSSI et al., as regards the preparation of the virus in T lymphocyte cultures derived either from blood, or from the umbilical cord, or also from bone marrow cells of adult donors in good health. This process comprises particularly the following essential steps :

- producing a viral infection of these T lymphocytes, after activation by a lectin mitogen, with a viral suspension derived from a crude supernatant liquor of lymphocytes producing the virus (initially obtained from a patient infected with AIDS or LAS),
- culturing cells infected with TCGF, in the presence of anti-$\alpha$-interferon sheep serum,
- effecting purification of the virus produced (production starts generally between the 9th and the 15th day following infection and lasts from 10 to 15 days), which purification comprises precipitating the virus in polyethyleneglycol in order to produce a first concentration of the virus, then centrifuging the preparation obtained in a 20-60 % sucrose gradient or in an isotonic gradient of metrizanide (sold under the trade mark NYCODENZ® by NYEGAARD, Oslo) and recovering the virus with the band having a density of 1.16-1.17 in the sucrose gradient or of 1.10-1.11 in the NYCODENZ® gradient.

The LAV virus may also be produced from permanent cell lines of type T, such as the CEM line, or from B lymphoblastoid cell lines, such as obtained by the transformation of the lymphocytes derived from a healthy donor with the Epstein-Barr virus, for instance as disclosed in EP-A- 0 165 120. The permanent cell lines obtained produce continuously a virus (designated as LAV-B in the case of the B lymphoblastoïd cell lines) which possesses the essential antigenic and morphological features of the LAV viruses (except that it is collected in a density band sometimes slightly higher than in the preceding case (particularly 1.18) in sucrose. The final purification of the virus can also be carried out in a NYCODENZ gradient.

Reference is made to the article of Schüpbach et al, Science. vol. 224, pages 503-505, 4 may 1984. This article discloses a work with respect to different antigens of the HTLV-III retrovirus.

Results are presented including a reference to an antigen having migrated on an electrophoresis gel at a migration distance corresponding to a molecular weight of approximately 110,000.

Schüpbach et al mentioned that this antigen was detected in a virus preparation but was below limit of detection in the cells.

A method for cloning DNA sequences hybridizable with the genomic RNA of LAV has already been disclosed in EP-A- 0 178 978. Reference is hereafter made to that application as concerns the subject matter in common with the further improvements to the invention disclosed herein.

The present invention further aims at providing additional new means which should not only also be useful for the detection of LAV or related viruses (hereafter more generally referred to as "LAV viruses"), but also have more versatility, particularly in detecting specific parts of the genomic DNA of said viruses whose expression products are not always directly detectable by immunological methods. The present invention further aims at providing polypeptides containing sequences in common with polypeptides comprising antigenic determinants included in the proteins encoded and expressed by the LAV genome occurring in nature. An additional object of the invention is to further provide means for the detection of proteins related to LAV viruses, particularly for the diagnosis of AIDS or pre-AIDS or, to the contrary, for the detection of antibodies against the LAV viruses or proteins related therewith, particularly in patients afflicted with AIDS or pre-AIDS or more generally in asymptomatic carriers and in blood-related products. Finally the invention also aims at providing immunogenic polypeptides, and more particularly protective polypeptides for use in the preparation of vaccine compositions against AIDS or related syndromes.

The present invention relates to additional DNA fragments, hybridizable with the genomic RNA of LAV as they will be disclosed hereafter, as well as with additional cDNA variants corresponding to the whole genomes of LAV viruses. It further relates to DNA recombinants containing said DNAs or cDNA fragments.

An unaltered purified LAV retrovirus distinguishes from those which have been defined above, in that it includes an amount of one or several envelope antigens, sufficient to be visualized when the virus is labelled with $^{35}$S-cysteine, free of unlabelled cysteine in a proportion of 200 microcuries per ml of medium. these antigens, among which particularly glycoproteins, are recognised selectively in vitro by serums of patients affected with AIDS or LAS or by the sera of asymptomatic carriers of the virus.

A preferred antigen according to the preceding definition obtainable from a lysate of this virus (or by gentle scouring of the envelope of the virus) is a glycoprotein having a molecular weight of the order of 110,000 daltons, as determined by its migration distance in comparison with the distances of migrations, in a same migration system, of standard proteins having known molecular weights. Particularly comparative measurements were made on a 12.5 % polyacrylamide gel under a voltage of 18 V for 18 hours, upon using the following standard proteins (marketed by AMERSHAM) :
- lysozyme-($^{14}$C)-methyl (MW: 14,300),
- carbon dioxide-($^{14}$C)-methyl (MW: 30,000),
- ovalbumin-($^{14}$C)-methyl (MW: 46,000),
- bovin albumin serum ($^{14}$C)-methyl (MW: 69,000),
- phosphorylase b-($^{14}$C)-methyl (MW: 92,500),
- myosine-($^{14}$C)-methyl (MW: 200,000).

The invention relates also to the antigens themselves, particularly that of molecular weight of about 110,000-120,000, which possess also the capability of being recognised by sera of patients suffering from AIDS or LAS or by serums of persons who have been exposed to LAV viruses or those analogous with the latter. These antigens have also the characteristic of forming complexes with concanavalin A, said complex being dissociatable in the presence of O-methyl-$\alpha$-D-mannopyranoside. The antigens according to the invention can also bind to other lectins for example those known under the name "LENTYL-LECTIN". The preferred antigen according to the invention, of molecular weight 110,000, is also sensitive to the action of endoglycosidases. This action is manifested by the production from the antigen of molecular weight 110,000 of a protein having a molecular weight of the order of 90,000, the latter being separable for example by immunoprecipitation or by separation employing the differences in molecular weights (migrations differentiated on gel).

Preferred antigens of the invention are constituted by glycoproteins.

The invention relates also to the process for producing the viruses according to the invention. This process distinguishes essentially from those recalled above at the level of the final purification operation. In particular, the purification step of the process according to the invention is no longer carried out in gradients, but involves the performance of differential centrifugations effected directly on the supernatants of the culture media of the producing cells. These centrifugation operations comprise particularly a first centrifugation at an angular centrifugation velocity, particularly of 10,000 rpm, enabling the removal of non-viral constituents, more particularly of cellular constituents, then a second centrifugation at higher angular velocity, particularly at 45,000 rpm, to obtain the precipitation of the virus itself. In preferred embodiments, the first centrifugation at 10,000 rpm, is maintained for 10 minutes and the second at 45,000 rpm, for 20 minutes. These are, of course, only indicative values, it being understood that it remains within the ability of the specialist to modify the centrifugation conditions, to provide for the separation of the cellular constituents and of the viral constituents.

This modification of the purification process results in the production of viral preparations from which the antigen mentioned can then be isolated more easily, than from virus preparations purified by the previous methods. In any event, the viruses finally obtained by the process of the present invention are more easily recognised by sera of patients or of persons who have been exposed to the LAV viruses or to morphologically and antigenically similar strains.

The antigens according to the invention can themselves be obtained from the above disclosed viruses, by lysis (or other suitable processing) of the latter in the presence of any suitable detergent and by recovery and separation of the antigens released. Advantageously, the lysis of the virus is effected in the presence of aprotinin or of any other agent suitable for inhibiting the action of proteases. The separation of the antigens according to the invention can then be carried out by any method known in itself ; for example, it is possible to proceed with a separation of the proteins by employing their respectively different migrations in a predetermined gel, the protein sought being then isolated from the zone of the gel in which it would normally be found in an electrophoresis operation under well determined conditions, having regard to its molecular weight. The antigens according to the invention can however be separated from the lysate of the abovesaid viruses, due to their affinity for lectins, in particular concanavaline A or lentyl-lectin. The lectin used is preferably immobilised on a solid support, such as the cross linked polymer derived from agarose and marketed under the trade mark SEPHAROSE. After washing of the fixed antigens with a suitable buffer, the antigens can be eluted in any suitable manner, particularly by resorting to a O-methyl-$\alpha$-D-mannopyranoside in solution.

A more thorough purification of these antigens can be performed by immunoprecipitation with the sera of patients known to possess antibodies effective against said protein, with concentrated antibody preparations (polyclonal antibodies) or again with monoclonal antibodies, more particularly directed against the antigen according to the invention, in particular that having the molecular weight of 110,000, denoted below by the abbreviation gp110.

Additional characteristics of the invention will appear also in the course of the description, which follows, of the isolation of a virus according to the invention and of antigens, particularly an envelope antigen of the virus. Reference will be made to the drawings in which :

Figure 1 is derived from a photographic reproduction of gel strips which have been used to carry out electrophoreses of lysate extracts of T lymphocytes, respectively infected and uninfected (controls) by a LAV suspension.

Figures 2 and 3 are the restriction map of a complete LAV genome (clone $\lambda$J19) or of fragments thereof.

Figures 4a to 4e are the complete sequence of a LAV viral genome.

Figures 5 and 6 show diagrammatically parts of the three possible reading phases of LAV genomic RNA, including the open reading frames (ORF) apparent in each of said reading phases.

## I - PRODUCTION OF THE VIRUS AND OF ANTIGENS

T lymphocytes derived from a healthy donor and infected with LAV1, under the conditions described by F. BARRE-SINOUSSI et Coll., on CEM cells derived from a patient afflicted with leukemia and also infected in vitro with LAV1, were kept under cultivation in a medium containing 200 microcuries of $^{35}$S-cystein and devoid of unlabelled cystein. The infected lymphocytes were cultured in a non denaturing medium to prevent the degradation of the antigen sought. The supernatant liquor from the culture medium was then subjected to a first centrifugation at 10,000 rpm for 10 minutes to remove the non viral components, then to a second centrifugation at 45,000 rpm for 20 minutes for sedimenting the virus. The virus pellet was then lysed by detergent in the presence of aprotinin (5 %) particularly under the conditions described in the article of F. BARRE-SINOUSSI et Coll.

The same operation was repeated on lymphocytes taken up from a healthy donor as control.

The various lysates were then immuno-precipitated by sera of patients suffering from AIDS or with LAS. Sera originating from healthy donors or of donors infected with other diseases were immunoprecipitated too. The media were then subjected to electrophoreses in a SDS-polyacrylamide gel.

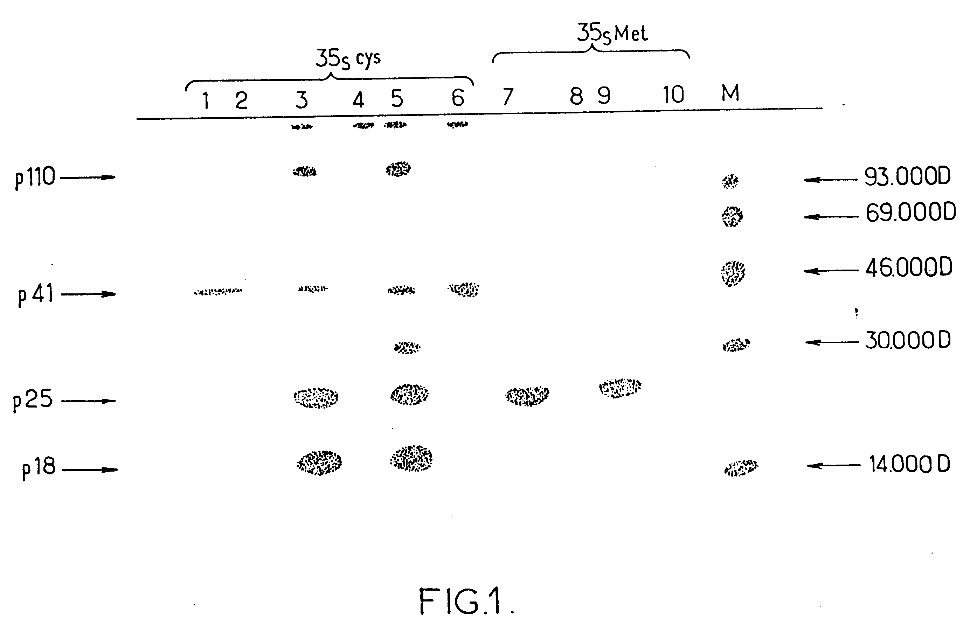

The results are indicated in figure 1. The gel strips numbered from 1 to 6 were obtained from preparations labelled by $^{35}$S-cysteine. The strips numbered 7 to 10 show results observed on infected or uninfected lymphocyte preparations labelled with $^{35}$S-methionine. Finally the strip M corresponds to the migration distances of the standard proteins identified above, whose molecular weights are recalled in the right hand portion of the figure.

The references to the labelled viral proteins appear on the left handside of the figure.

It is noted that columns 7 and 9 show the specific protein p25 of LAV, labelled with $^{35}$S-methionin. The same protein is absent on strips 8 and 10 corresponding to results obtained with a preparation originating from healthy lymphocytes.

4

Columns 3 and 5 correspond to the results which have been obtained with preparations from lymphocytes infected and labelled with $^{35}$S-cysteine. The proteins p25 and p18, the characteristic core proteins of LAV, and the glycoprotein gp110, also specific of LAV, were also present. Images corresponding to a protein p41 (molecular weight of the order of 41,000) appeared in the various preparations, although less distinctly.

The virus according to the invention and the antigen according to the invention can be either precipitated by lectins, particularly concanavalin A, or fixed to a SEPHAROSE-concanavalin A column. Particularly the purification of the envelope glycoproteins can be carried out as follows. This fixation can particularly be carried out by contacting a lysate of the LAV virus dissolved in a suitable buffer with concanavalin-A bound to SEPHAROSE®. A suitable buffer has the following composition :

Tris       10 mM
NaCl       0.15 M
CaCl$_2$     1 mM
MgCl$_2$     1 mM
Detergent marketed under the trade mark TRITON 1 %
pH       7.4

When the fixation has been achieved, the SEPHAROSE®-concanavalin A is washed with a buffer of the same composition, except that the TRITON® concentration is lowered to 0.1 %. The elution is then effected with an 0.2 M O-methyl-$\alpha$-D-mannopyranoside solution in the washing buffer.

The protein may be further concentrated by immuno-precipitation with antibodies contained in the sera of patients infected with AIDS virus or with polyclonal antibodies obtained from a serum derived from an animal previously immunised against the "unaltered" virus according to the invention or the abovesaid glycoprotein. The protein can then be recovered by dissociation of the complex by a solution having an adequate content of ionic salt. Preferably the antibody preparation is itself immobilised in a manner known in itself on an insoluble support, for instance of the SEPHAROSE® B type.

It is also possible to resort to monoclonal antibodies secreted by hybridomas previously prepared against gp 110. These monoclonal antibodies, as well as the hybridomas which produce them, also form part of the invention.

A technique for producing and selecting monoclonal antibodies directed against the gp110 glycoprotein is described below.

## Immunisation of the mice

Groups of Balb/c mice from 6 to 8 weeks old were used. One group receives the virus carrying the abovesaid glycoprotein, another a purified glycoprotein gp110. The immunisation procedure, identical for all mice, comprises injecting 10 mg of the antigenic preparation in the presence of Freund's complete adjuvant at day 0, then again but in the presence of Freund's incomplete adjuvant at day 14 and without adjuvant at days 28 and 42. The three first injections are made intraperitoneally, the fourth intravenously.

## Fusion and culture of the hybrids

The non secreting myeloma variant 5.53 P3 x 63 Ag8, resistant to azaguanine, itself derived from the MOPC-21 cell-line, is used. Fusion with immunised mouse splenocytes is carried out in the presence of polyethylene-glycol 4000 by the technique of FAZEKAS de st-GROTH and SCHEIDEGGER on the 45th day. The selection of the hybrids in RPMI 16-40 "HAT" medium is carried out in plates having 24 wells (known under the designation COSTAR) by resorting to the same culture techniques.

The hybridomas producing antibodies of adequate specificity are then cloned in plates having 96 wells, in the presence of a "feeder" layer of syngeneic thymocytes. The producing clones thus selected are then expanded in 24 wells plates, still in the presence of thymocytes. When the confluence appears in one of the wells, the clone is injected intraperitoneally into a balb/c mouse which had received an injection of PRISTANE 8 days previously and/or is kept in liquid culture.

## Demonstration of the anti-LAV antibodies

Five different techniques enable characterisation of the clones producing antibodies of suitable specificity. In a first stage, the hybrids producing antibodies are determined by an ELISA test revealing mouse immunoglobulins in the supernatant liquors. From this first selection, supernatants are sought which have antibodies directed against viral constituents by means of an ELISA test revealing anti-LAV antibodies, or by immunofluorescence on the virus producing human cells. Finally, the supernatant liquors are analysed by radio-immunoprecipitation of virus labelled with cystein and by the Western-Blot technique on a viral preparation which permit the determination of the specificities of these anti-LAV antibodies.

RESULTS

Cells obtained from the various fusions are placed under culture in 648 wells. Their microscopic examination shows that the majority of these wells contain a single hybrid clone capable of growing in a "HAT" selective medium. More than 50 % among them produce antibodies giving rise to a positive response under ELISA antivirus examination. The most representative fusions are tested by the Western-Blot technique and several of them are subcloned, taking into account their respective specificities reactivities in antivirus ELISA and their behaviours under the culturing conditions. Those hybrids which are more particularly selected are those which produce antibodies which selectively recognise the viral glycoprotein gp110 having a molecular weight of about 110 KD. All the sub clonings give rise to clones producing antibodies which, after expression, are injected into syngeneic mice. Analysis of the specificities of the antibodies present in the different ascites fluids confirms the specificity of the antibodies of said ascites with respect to gp110.

The monoclonal antibodies obtained can themselves be employed to purify proteins containing an antigenic site also contained in gp110. The invention relates therefore also to these processes of purification as such. This process is advantageously applied to virus lysates or T lymphocyte lysates or other cells producing LAV or the like, when care has been taken to avoid the uncontrolled separation of gp110 during the purification procedure of the virus, prior to lysis thereof. Needless to say that the process can also be applied to any solution containing gp110 or a protein, polypeptide or glycoprotein comprising an antigenic site normally carried by the envelope protein and recognised by the monoclonal antibody. For practising this process, the monoclonal antibodies are advantageously immobilised on a solid support, preferably adapted to affinity chromatography operations. For example, these monoclonal antibodies are fixed to an agarose lattice with three-dimensional cross-linking, marketed under the trade mark SEPHAROSE by the Swedish company PHARMACIA A.G., for example by the cyanogen bromide method.

The invention therefore also relates to a process for separating the antigens concerned, which process comprises contacting a mixture of antigens, including those of interest (for instance a virus lysate or extract), with an affinity column bearing the abovesaid monoclonal antibodies, to selectively fix polypeptides, proteins or glycoproteins selectively recognized by said monoclonal antibodies, recovering the latter by dissociation of the antigen-antibody complex by means of a suitable buffer, particularly a solution of adequate ionic strength, for example of a salt, preferably ammonium acetate (which leaves no residue upon freeze drying of the preparation) or a solution acidified to a pH 2-4 or to a glycine buffer at the same pH and recovering the eluted polypeptides, proteins or glycoproteins.

It is self-evident that the invention relates also to polypeptide fragments having lower molecular weights and carrying antigenic sites recognizable by the same monoclonal antibodies. It is clear to the specialist that the availability of monoclonal antibodies recognizing the gp110 glycoprotein gives also access to smaller peptide sequences or fragments containing the common antigenic site or epitope. Fragments of smaller sizes may be obtained by resorting to known techniques. For instance such a method comprises cleaving the original larger polypeptide by enzymes capable of cleaving it at specific sites. By way of examples of such proteins, may be mentioned the enzyme of Staphylococcyus aureus V8, $\alpha$-chymotrypsin, "mouse sub-maxillary gland protease" marketed by the BOEHRINGER company, Vibrio alginolyticus chemovar iophagus collagenase, which specifically recognises said peptides Gly-Pro and Gly-Ala, etc..

It is also possible to obtain polypeptides or fragments of envelope antigens of the virus, by cloning fragments excised from a cDNA constructed from genomes of LAV variants.

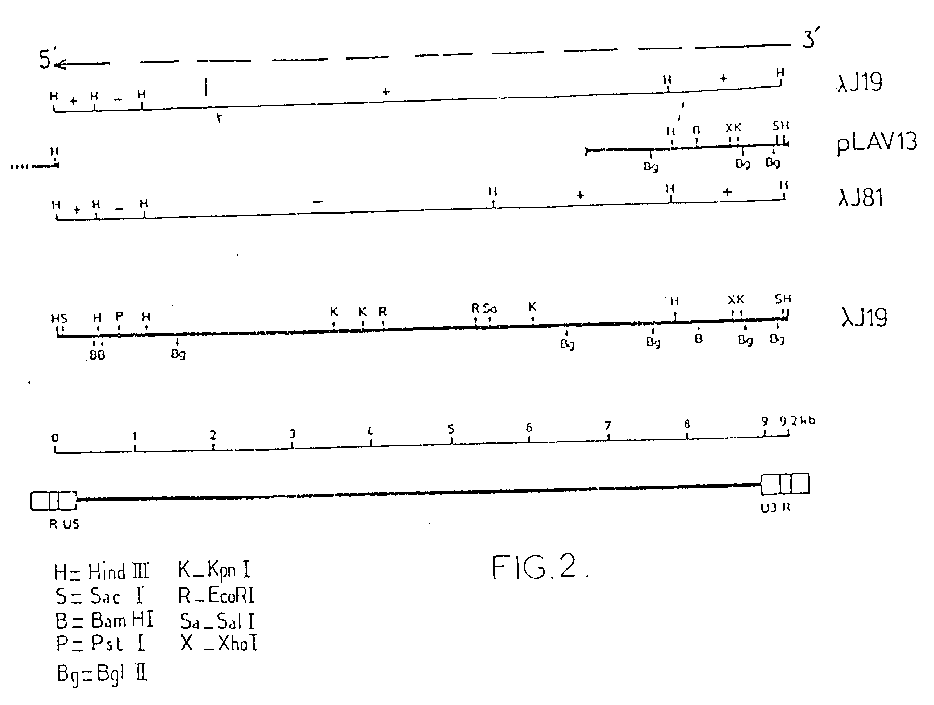

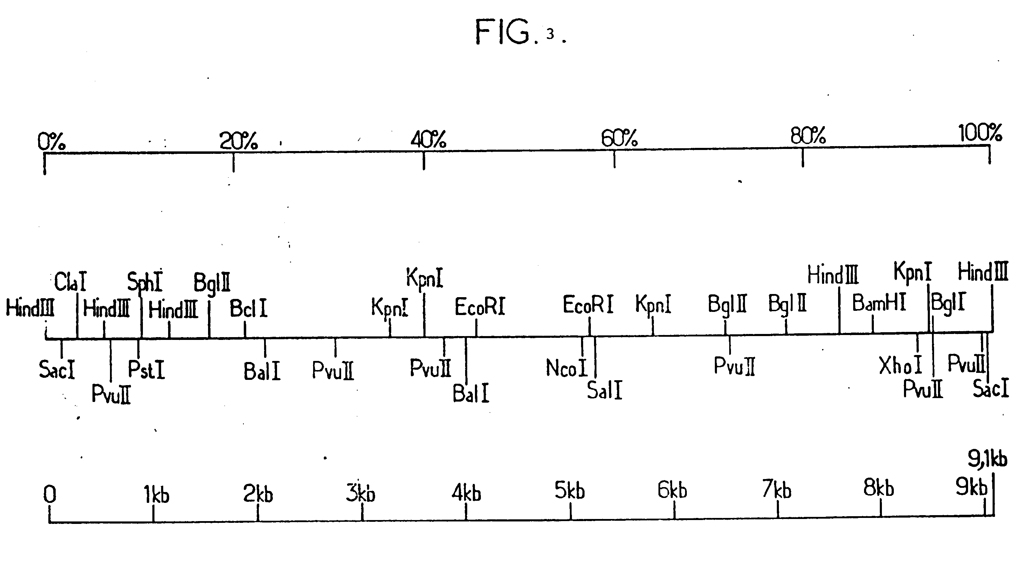

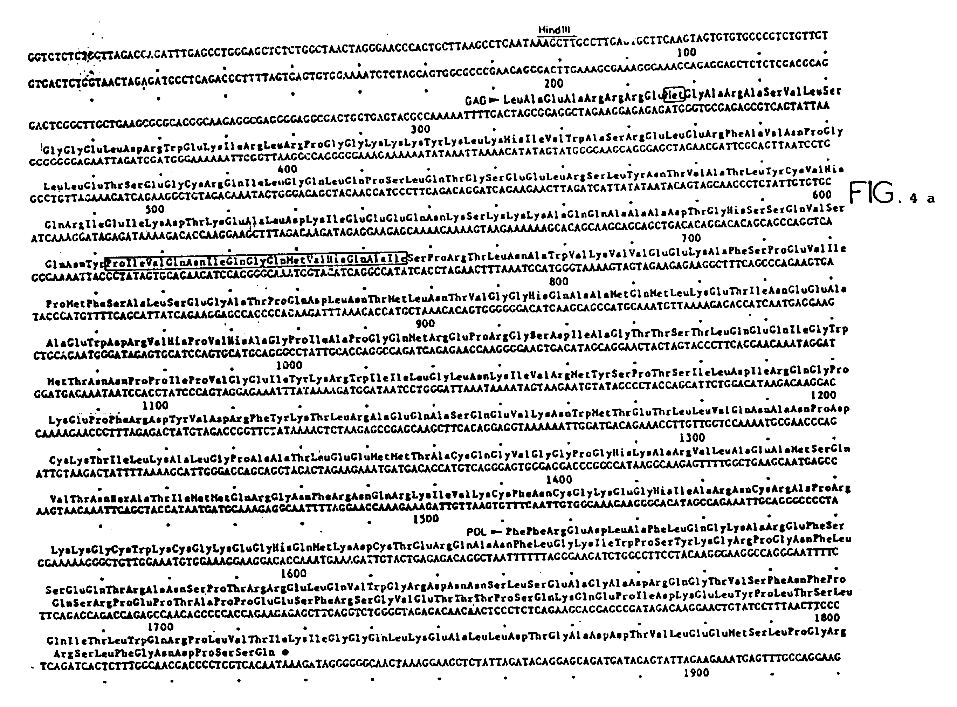

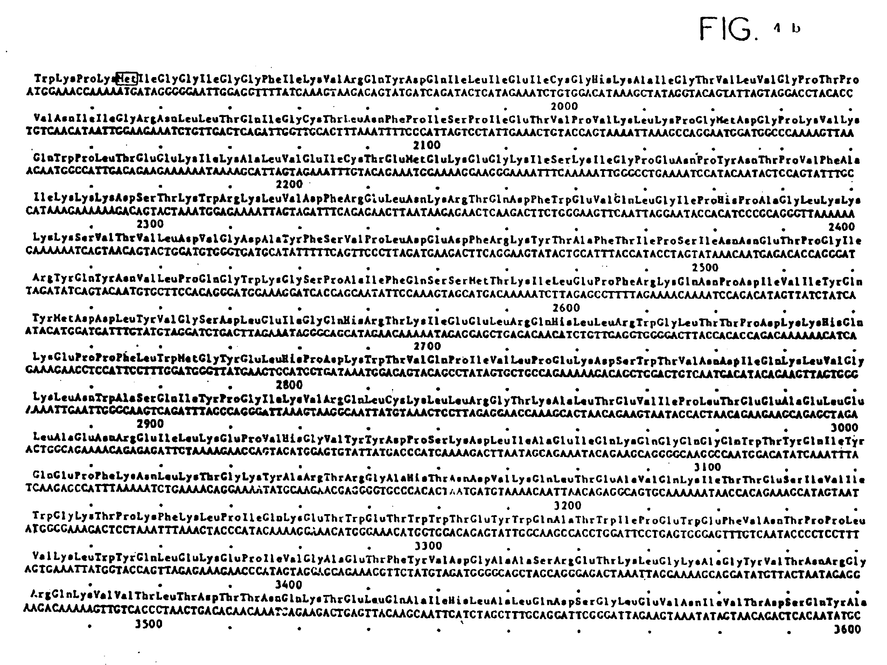

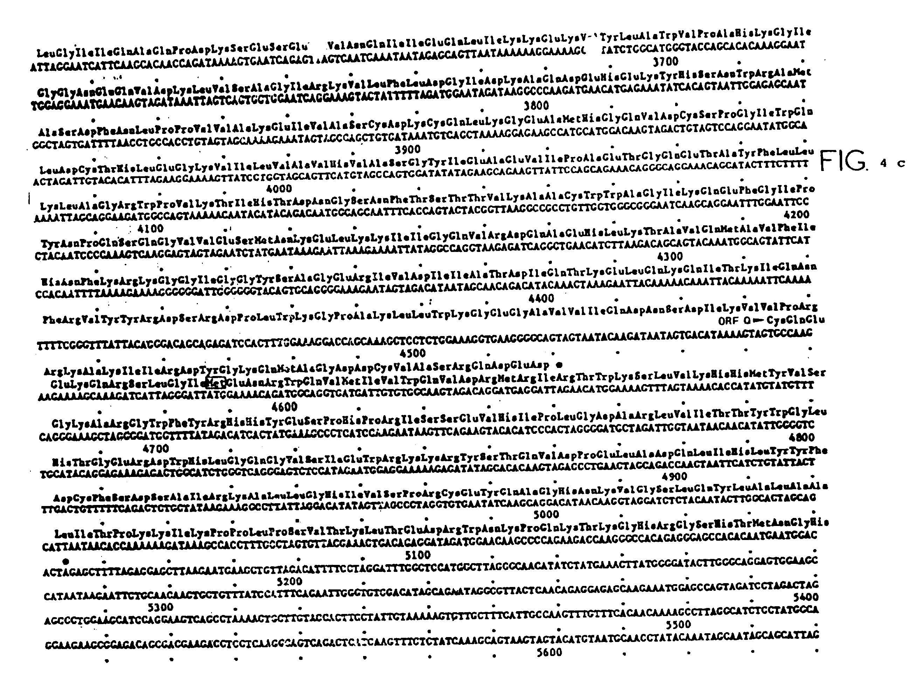

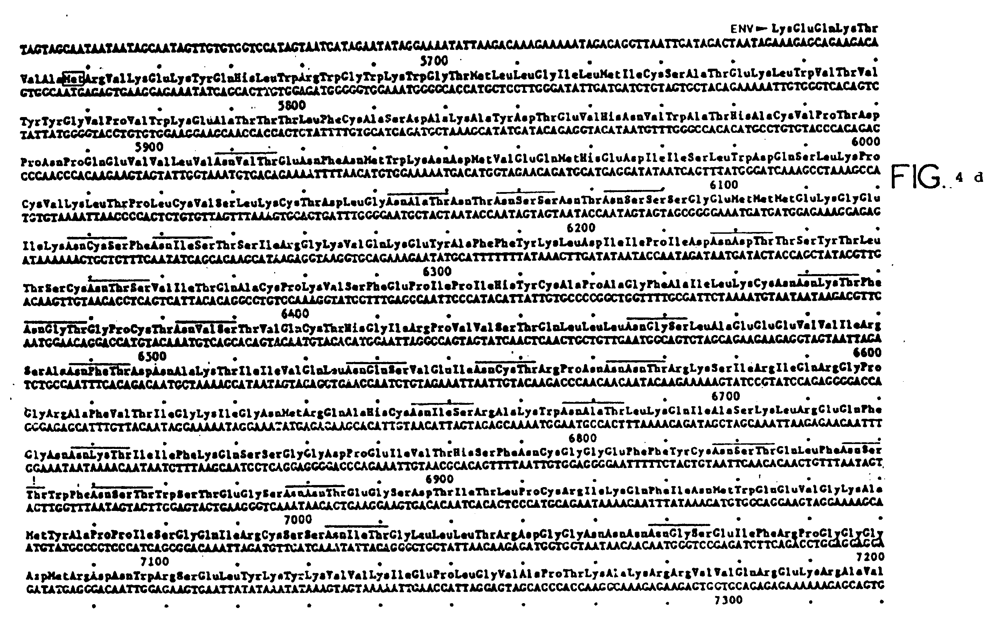

Figures 2 and 3 are restriction maps of such a cDNA comprising a total of 9.1 to 9.2 kb. The polypeptides encoded by cDNA fragments are located in the region extending between site KpnI (position 6100) and site BglII (position 9150) of the restriction map of Figure 2. The presence of a characteristic site of an envelope antigen of the LAV virus or the like in any polypeptide expressed (in a suitable host cell transformed beforehand by a corresponding fragment or by a vector containing said fragment) can be detected by any suitable immunochemical means.

The invention relates more particularly to polypeptides encoded by cDNA fragments defined hereafter. It also relates to the nucleic acid fragments themselves, including a cDNA variant corresponding to a whole LAV retroviral genome, characterized by a series of restriction sites in the order hereafter (from the 5′ end to the 3′ end).

The coordinates of the successive sites of the whole LAV genome (see also restriction map of λJ19 in fig. 2) are indicated hereafter too, with respect to the Hind III site (selected as of coordinate 1) which is located in the R region. The coordinates are estimated with an accuracy of ± 200 bp :

| Hind II | 0 |
|---|---|
| Sac I | 50 |
| Hind III | 520 |
| Pst I | 800 |
| Hind III | 1 100 |
| Bgl II | 1 500 |
| Kpn I | 3 500 |
| Kpn I | 3 900 |
| Eco RI | 4 100 |
| Eco RI | 5 300 |
| Sal I | 5 500 |
| Kpn I | 6 100 |
| Bgl II | 6 500 |
| Bgl II | 7 600 |
| Hind III | 7 850 |
| Bam HI | 8 150 |
| Xho I | 8 600 |
| Kpn I | 8 700 |
| Bgl II | 8 750 |
| Bgl II | 9 150 |
| Sac I | 9 200 |
| Hind III | 9 250 |

Another DNA variant according to this invention optionally contains an additional Hind III approximately at the 5 550 coordinate.

Reference is further made to fig. 1 which shows a more detailed restriction map of said whole-DNA (λJ19).

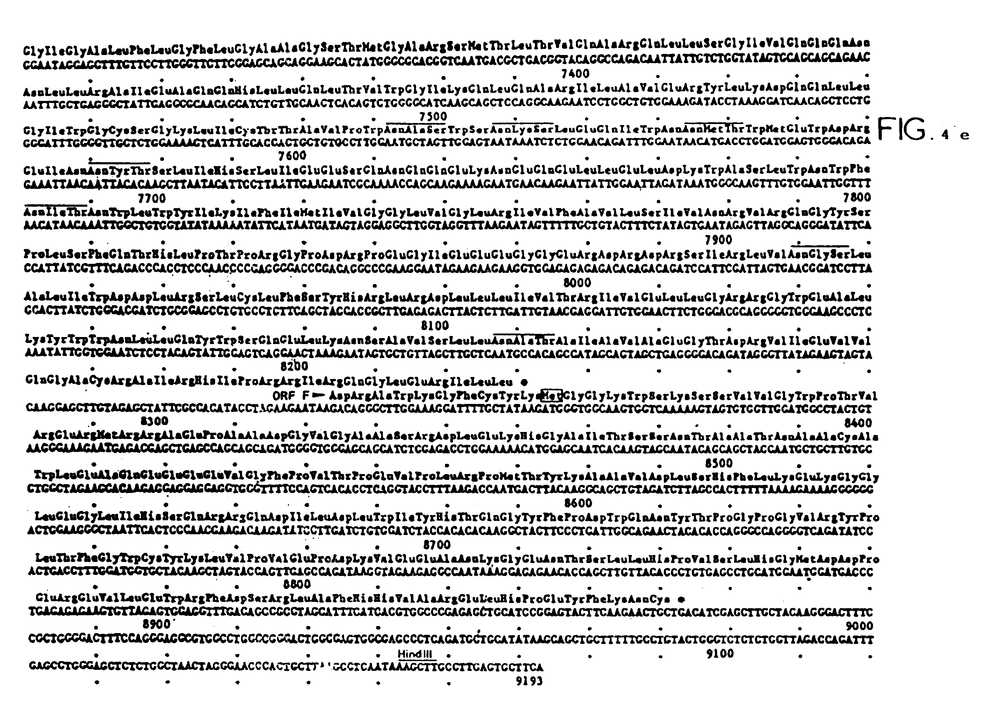

An even more detailed nucleotide sequence of a preferred DNA according to the invention is shown in figs. 4a-4e hereafter.

The invention further relates to other preferred DNA fragments and polypeptide sequences (glycosylated or not glycosylated) which will be referred to hereafter.

## SEQUENCING OF LAV

The sequencing and determination of sites of particular interest were carried out on a phage recombinant corresponding to λJ19 disclosed in the abovesaid EP 0 178 978. A method for preparing it is disclosed in that application.

The whole recombinant phage DNA of clone λJ19 (disclosed in the earlier application) was sonicated according to the protocol of DEININGER (1983), Analytical Biochem. 129, 216. The DNA was repaired by a Klenow reaction for 12 hours at 16°C. The DNA was electrophoresed through 0.8 % agarose gel and DNA in the size range of 300-600 bp was cut out and electroeluted and precipitated. Resuspended DNA (in 10 mM Tris, pH 8 ; 0,1 mM EDTA) was ligated into M13mp8 RF DNA (cut by the restriction enzyme SmaI and subsequently alkaline phosphated), using T4 DNA- and RNA-ligases (Maniatis T et al (1982) - Molecular cloning - Cold Spring Harbor Laboratory). An E. coli strain designated as TG1 was used for further study. This strain has the following genotype :

Δlac pro, supE, thi.F′traD36, proAB, lacIq, ZΔM15,r⁻

This E. coli TGI strain has the peculiarity of enabling recombinants to be recognized easily. The blue colour of the cells transfected with plasmids which did not recombine with a fragment of LAV DNA is not modified. To the contrary cells transfected by a recombinant plasmid containing a LAV DNA fragment yield white colonies. The technique which was used is disclosed in Gene (1983), 26, 101.

This strain was transformed with the ligation mix using the Hanahan method (Hanahan D (1983) J. Mol. Biol. 166, 557). Cells were plated out on a tryptone-agarose plate with IPTG and X-gal in soft agarose. White plaques were either picked and screened or screened directly in situ using nitrocellulose filters. Their DNAs were hybridized with nick-translated DNA inserts of pUC18 Hind III subclones of λJ19. This permitted the isolation of the plasmids or subclones of λ which are identified in the table hereafter. In relation to this table it should also be noted that the designation of each plasmid is followed by the deposition number of a cell culture of E. coli TGI containing the corresponding plasmid at the "Collection Nationale des Cultures de Micro-organismes" (C.N.C.M.) of the Pasteur Institute in Paris, France. A non-transformed TGI cell line was also deposited at the C.N.C.M. under Nr. I-364. All these deposits took place on November 15, 1984. The sizes of the corresponding inserts derived from the LAV genome have also been indicated.

## TABLE

### Essential features of the recombinant plasmids

- pJ19 - 1 plasmid    (I-365)    0.5 kb

    Hind III - Sac I - Hind III

- pJ19 - 17 plasmid    (I-367)    0.6 kb

    Hind III - Pst 1 - Hind III

- pJ19 - 6 plasmid    (I-366)    1.5 kb

    Hind III (5')
    Bam HI
    Xho I
    Kpn I
    Bgl II
    Sac I (3')
    Hind III

- pJ19-13 plasmid    (I-368)    6.7 kb

    Hind III (5')
    Bgl II
    Kpn I
    Kpn I
    Eco RI
    Eco RI
    Sal I
    Kpn I
    Bgl II
    Bgl II
    Hind III (3')

Positively hybridizing M13 phage plates were grown up for 5 hours and the single-stranded DNAs were extracted.

M13mp8 subclones of λJ19 DNAs were sequenced according to the dideoxy method and technology devised by Sanger et al (Sanger et al (1977), Proc. Natl. Acad. Sci. USA, 74 , 5463) and M13 cloning and sequencing handbook, AMERSHAM (1983). The 17-mer oligonucleotide primer $\alpha$-$^{35}$SdATP (400Ci/mmol, AMERSHAM), and 0.5X-5X buffer gradient gels (Biggen M.D. et al (1983, Proc. Natl. Acad. Sci. USA, 50, 3963) were used. Gels were read and put into the computer under the programs of Staden (Staden R. (1982), Nucl. Acids Res. 10, 4731). All the appropriate references and methods can be found in the AMERSHAM M13 cloning and sequencing handbook.

The complete DNA sequence of λJ19 (also designated as LAV-Ia) is shown in figs. 4 to 4e.

The sequence was reconstructed from the sequence of phage λJ19 insert. The numbering starts at the cap site which was located experimentally (see hereafter). Important genetic elements, major open reading frames and their predicted products are indicated together with the HindIII cloning sites. The potential glycosylation sites in the env gene are overlined. The $NH_2$-terminal sequence of $p25^{gag}$ determined by protein microsequencing is boxed.

Each nucleotide was sequenced on average 5.3 times : 85 % of the sequence was determined on both strands and the remainder sequenced at least twice from independent clones. The base composition is T, 22.2 % ; C, 17.8 % ; A, 35.8 % ; G, 24.2 % ; G + C, 42 %. The dinucleotide GC is greatly under represented (0.9 %) as is common amongst eukaryotic sequences (Bird 1980).

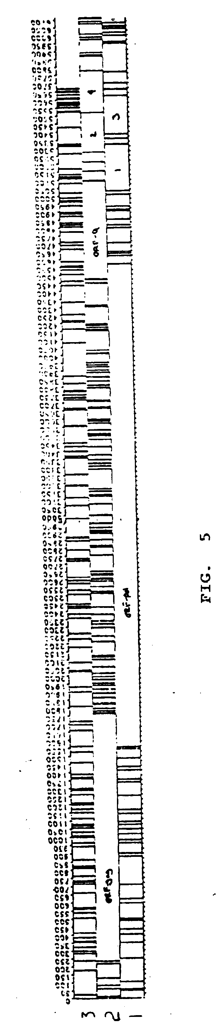

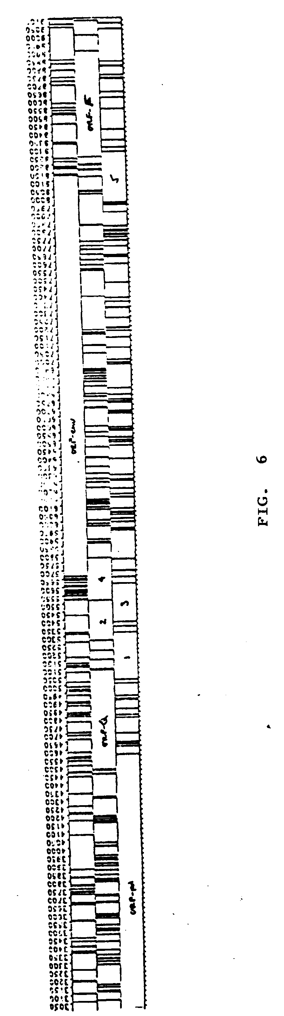

Figs. 5 and 6 provide a diagrammatized representation of the lengths of the successive open reading frames corresponding to the successive reading phases (also referred to by numbers "1", "2" and "3" appearing in the left handside part of fig. 5). The relative positions of these open reading frames (ORF) with respect to the nucleotidic structure of the LAV genome is referred to by the scale of numbers representative of the respective positions of the corresponding nucleotides in the DNA sequence. The vertical bars correspond to the positions of the corresponding stop codons.

The following genes and DNA fragments can be distinguished on the different reading frames shown. Reference is then also made to the proteins or glycoproteins encoded by said genes and fragments particularly to ORFs sequences such as ORF-Q, ORF-F.

The invention further concerns DNA sequences which provide open reading frames defined as ORF-Q, ORF-F and as "1", "2", "3", "4", "5", the relative positions of which appear more particularly in figs. 2 and 3.

These ORFs have the following locations :

| ORF-Q | phase 1 | start 4554 | stop 5162 |
|-------|---------|------------|-----------|
| ORF-F | " 2 | " 8324 | " 8972 |
| ORF-1 | " 1 | " 5105 | " 5392 |
| ORF-2 | " 2 | " 5349 | " 5591 |
| ORF-3 | " 1 | " 5459 | " 5692 |
| ORF-4 | " 2 | " 5595 | " 5849 |
| ORF-5 | " 1 | " 8042 | " 8355 |

ORFs Q and F

The viral (+) strand of the LAV genome was found to contain the statutory retroviral genes encoding the core structural proteins (gag), reverse transcriptase (pol) and envelope protein (env), and two extra open reading frames (orf) which we call Q and F (Table 1). The genetic organization of LAV, 5'LTR-gag-pol-Q-env-F-3'LTR, is unique. Whereas in all replication competent retrovisruses pol and env genes overlap, in LAV they are separated by orf Q (192 amino acids) followed by four small (<100 triplets) orf. The orf F (206 amino acids) slightly overlaps the 3' end of env and is remarkable in that it is half encoded by the U3 region of the LTR.

Such a structure places LAV clearly apart from previously sequenced retroviruses (Fig. 2). The (-) strand is apparently non coding. The additional HindIII site of the LAV clone λJ81 (with respect to λJ19) maps to the apparently non-coding region between Q and env (positions 5166-5745). Starting at position 5501 is a sequence (AAGCCT) which differs by a single base (underlined) from the HindIII recognition sequence. It is to be anticipated that many of the restriction site polymorphism between different isolates will map to this region. Clone λJ81 has also been referred to in British application Nr. 84 23659 filed on September 15, 1984.

Q and F :

The nucleotide positions of their respective extremities are given in Table 1 hereafter.

The location of orf Q is without precedent in the structure of retroviruses. Orf F is unique in that it is half encoded by the U3 element of the LTR. Both orfs have "strong" initiator codons (Kozak 1984) near their 5'

ends and can encode proteins of 192 aminoacids (MW calc = 22487) and 206 aminoacids (MWcalc = 23316) respectively. Both putative proteins are hydrophilic (pQ 49 % polar, 15.1 % Arg + Lys ; pF 46 % polar, 11 % Arg + Lys) and are therefore unlikely to be associated directly with membrane. The function for the putative proteins pQ and pF cannot be predicted as no homology was found by screening protein sequence data bases. Between orf F and the pX protein of HTLV-1 there is no detectable homology. Furthermore their hydrophobicity/hydrophilicity profiles are completely different. It is known that retroviruses can transduce cellular genes notably proto-oncogenes (Weinberg 1982)). We suggest that orfs Q and F represent exogenous genetic material and not some vestige of cellular DNA because (I) LAV DNA does not hybridize to the human genome under stringent conditions (Alizon et al., 1984), (II) their codon usage is comparable to that of the gag, pol and env genes (data not shown).

The organization of a reconstructed LTR and viral flanking elements are shown schematically in Fig. 6. The LTR is 638 bp long and displays usual features (Chen and Barker 1984) : (I) It is bounded by an inverted repeat (5'ACTG) including the conserved TG dinucleotide (Temin 1981), (II) Adjacent to 5' LTR is the tRNA primer binding site (PBS), complementary to $tRNA_3^{lys}$ (Raba et al., 1979), (III) adjacent to 3'LTR is a perfect 15 bp polypurine tract. The other three polypurine tracts observed between nucleotides 8200-8800 are not followed by a sequence which is complementary to that just preceding the PBS. The limits of U5, R and U3 elements were determined as follows. U5 is located between PBS and the polyadenylation site established from the sequence of the 3' end of oligo(dT)-primed LAVcDNA (Alizon et al., 1984). Thus U5 is 84 bps long. The length of R+U5 was determined by synthesizing tRNA-primed LAV cDNA. After alkaline hydrolysis of the primer, R+U5 was found to be 181 ± 1 bps. Thus A is 97 bps long and the capping site at its 5' end can be located. Finally U3 is 456 bp long. The LAV LTR also contains characteristic regulatory elements : a polyadenylation signal sequence AATAAA 19 bps from the R-U5 junction and the sequence ATATAAG which is very likely the TATA box, 22 bps 5' of the cap site. There are no longer direct repeats within the LTR. Interestingly the LAV LTR shows some similarities to that of the mouse mammary tumour virus (MMTV) Donehower et al., 1981). They both use $tRNA_3^{lys}$ as a primer for (-) strand synthesis whereas all other exogenous mammalian retroviruses known to date use tRNA[pro] (Chen and Barker 1984). They possess very similar polypurine tracts (that of LAV is AAAAGAAAAGGGGGG while that of MMTV is AAAAAAGAAAAAGGGGG). It is probable that the viral (+) strand synthesis is discontinuous since the polypurine tract flanking the U3 element of the 3' LTR is found exactly duplicated in the 3' end of orf pol, at 4331-4336. In addition, MMTV and LAV are exceptionnal in that the U3 element can encode an orf. In the case of MMTV, U3 contains the whole orf while in LAV, U3 contains 110 codons of the 3' half of orf F.

The LAV long terminal repeat (LTR) is diagrammatically represented in Fig. 6. As mentioned the LTR was reconstructed from the sequence of λJ19 by juxtaposing the sequences adjacent to the HindIII cloning sites.

Sequencing of oligo(dT) primed LAV DNA clone pLAV75 (Alizon et al., 1984) rules out the possibility of clustered HindIII sites in the R region of LAV. LTR are limited by an invertd repeat sequence (IR). Both of the viral elements flanking the LTR have been represented = tRNA primer binding site (PBS) for 5' LTR and polypurine track (PU) for 3' LTR. Also indicated are a putative TATA box, the cap site, polydenylation signal (AATAAA) and polyadenylation site (CAA). The location of the open reading frame F (648 nucleotides) is shown above the LTR schema.

The LTRs (long terminal repeats) can also be defined as lying between position 8560 and position 160 (end extending over position 9097/1). As a matter of fact the end of the genome is at 9097 and, because of the LTR structure of the retrovirus, links up with the beginning of the sequence :

<div align="center">

Hind III

CTCAATAAAGCTTGCCTTG

9097  1

</div>

Table 1 sums up the locations and sizes of viral open reading frames. The nucleotide coordinates refer to the first base of the first triplet (1st tripet), of the fir methionine initiation codon (Met) and of the stop codon (stop). The number of aminoacids and calculated molecular weights are those calculated for unmodified precursor products starting at the first methionine through to the end with the exception of pol where the size and MW refer to those of the whole orf.

| orf | 1st triplet | Met | stop | No amino acids | MWcalc |
|-----|-----|-----|-----|-----|-----|
| gag | 312 | 336 | 1836 | 500 | 55841 |
| pol | 1631 | 1934 | 4640 | (1003) | (113629) |
| orf Q | 4554 | 4587 | 5163 | 192 | 22487 |
| env | 5746 | 5767 | 8350 | 861 | 97376 |
| orf F | 8324 | 8354 | 8972 | 206 | 23316 |

Table 1 : Location and sizes of viral open reading frames.

The invention concerns more particularly all the DNA fragments which have been more specifically referred to hereabove and which correspond to open reading frames. It will be understood that the man skilled in the art will be able to obtain them all, for instance by cleaving an entire DNA corresponding to the complete genome of a LAV species, such as by cleavage by a partial or complete digestion thereof with a suitable restriction enzyme and by the subsequent recovery of the relevant fragments. The different DNAs disclosed above can be resorted to also as a source of suitable fragments. The techniques disclosed hereafter for the isolation of the fragments which were then included in the plasmids referred to hereabove and which were then used for the DNA sequencing can be used.

Of course other methods can be used. Some of them have been examplified in EP-A- 0 178 978. Reference is for instance made to the following methods:

a) DNA can be transfected into mammalian cells with appropriate selection markers by a variety of techniques: calcium phosphate precipitation, polyethylene glycol, protoplast-fusion, etc..

b) DNA fragments corresponding to genes can be cloned into expression vectors for E. coli , yeast- or mammalian cells and the resultant proteins purified.

c) The proviral DNA can be "shot-gunned" (fragmented) into prokaryotic expression vectors to generate fusion polypeptides. Recombinant producing antigenically competent fusion proteins can be identified by simply screening the recombinants with antibodies against LAV antigens .

The invention further refers more specifically to DNA recombinants, particularly modified vectors, including any of the preceding DNA sequences and adapted to transform corresponding microorganisms or cells, particularly eukaryotic cells such as yeasts, for instance Saccharomyces cerevisiae, or higher eucaryotic cells, particularly cells of mammals, and to permit expression of said DNA sequences in the corresponding microorganisms or cells.

More particularly the invention relates to such modified DNA recombinant vectors modified by the abovesaid DNA sequences and which are capable of transforming higher eukaryotic cells particularly mammalian cells. Preferably any of the abovesaid sequences are placed under the direct control of a promoter contained in said vectors and which is recognized by the polymerases of said cells, such that the first nucleotide codons expressed correspond to the first triplets of the above-defined DNA-sequences. Accordingly this invention also relates to the corresponding DNA fragments which can be obtained from LAV genomes or corresponding cDNAs by any appropriate method. For instance such a method comprises cleaving said LAV genomes or cDNAs by restriction enzymes preferably at the level of restriction sites surrounding said fragments and close to the opposite extremities respectively thereof, recovering and identifying the fragments sought according to sizes, if need be checking their restriction maps or nucleotide sequences (or by reaction with monoclonal antibodies specifically directed against epitopes carried by the polypeptides encoded by said DNA fragments), and further if need be, trimming the extremities of the fragment, for instance by an exonucleolytic enzyme such as Bal31, for the purpose of controlling the desired nucleotide-sequences of the extremities of said DNA fragments or, conversely, repairing said extremities with Klenow enzyme and possibly ligating the latter to synthetic polynucleotide fragments designed to permit the reconstitution of the nucleotide extremities of said fragments. Those fragments may then be inserted in any of said vectors for causing the expression of the corresponding polypeptide by the cell transformed therewith. The corresponding polypeptide can then be recovered from the transformed cells, if need be after lysis thereof, and purified, by methods such as electrophoresis. Needless to say that all conventionnal methods for performing these operations can be resorted to.

The invention also relates more specifically to cloned probes which can be made starting from any DNA fragment according to this invention, thus to recombinant DNAs containing such fragments, particularly any plasmids amplifiable in prokaryotic or eukaryotic cells and carrying said fragments.

Using the cloned DNA fragments as a molecular hybridization probe - either by labelling with radionucleotides or with fluorescent reagents - LAV virion RNA may be detected directly in the blood, body fluids and blood products (e.g. of the antihaemophylic factors such as Factor VIII concentrates) and vaccines, i.e. hepatitis B vaccine. It has already been shown that whole virus can be detected in culture supernatants of LAV producing cells. A suitable method for achieving that detection comprises immobilizing virus onto a support, e.g. nitrocellulose filters, etc., disrupting the virion and hybridizing with labelled (radiolabelled or "cold" fluorescent- or enzyme-labelled) probes. Such an approach has already been developed for Hepatitis B virus in peripheral blood (according to SCOTTO J. et al. Hepatology (1983), 3, 379-384).

Probes according to the invention can also be used for rapid screening of genomic DNA derived from the tissue of patients with LAV related symptoms, to see if the proviral DNA or RNA is present in host tissue and other tissues.

A method which can be used for such screening comprises the following steps : extraction of DNA from tissue, restriction enzyme cleavage of said DNA, electrophoresis of the fragments and Southern blotting of genomic DNA from tissues, subsequent hybridization with labelled cloned LAV proviral DNA. Hybridization in situ can also be used.

Lymphatic fluids and tissues and other non-lymphatic tissues of humans, primates and other mammalian species can also be screened to see if other evolutionary related retrovirus exist. The methods referred to hereabove can be used, although hybridization and washings would be done under non stringent conditions.

The DNAs or DNA fragments according to the invention can be used also for achieving the expression of LAV viral antigens for diagnostic purposes.

The invention relates generally to the polypeptides themselves, whether synthesized chemically isolated from viral preparation or expressed by the different DNAs of the invention, particularly by the ORFs or fragments thereof, in appropriate hosts, particularly prokaryotic or eukaryotic hosts, after transformation thereof with a suitable vector previously modified by the corresponding DNAs.

More generally, the invention also relates to any of the polypeptide fragments (or molecules, particularly glycoproteins having the same polypeptide backbone as the polypeptides mentioned hereabove) bearing an epitope characteristic of an LAV protein or glycoprotein, which polypeptide or molecule then has N-terminal and C-terminal extremities respectively either free or, independently from each other, covalently bound to aminoacids other than those which are normally associated with them in the larger polypeptides or glycoproteins of the LAV viruses, which last mentioned aminoacids are then free or belong to another polypeptide sequence. Particularly the invention relates to hybrid polypeptides containing any of the epitope-bearing-polypeptides which have been defined more specifically hereabove, recombined with other polypeptide fragments normally foreign to the LAV proteins, having sizes sufficient to provide for an increased immunogenicity of the epitope-bearing-polypeptide yet, said foreign polypeptide fragments either being immunogenically inert or not interfering with the immunogenic properties of the epitope-bearing-polypeptide.

Such hybrid polypeptides, which may contain up to 150, even 250 aminoacids, usually consist of the expression products of a vector which contained ab initio a nucleic acid sequence expressible under the control of a suitable promoter or replicon in a suitable host, which nucleic acid sequence had however beforehand been modified by insertion therein of a DNA sequence encoding said epitope-bearing-polypeptide.

Said epitope-bearing-polypeptides, particularly those whose N-terminal and C-terminal aminoacids are free, are also accessible by chemical synthesis, according to techniques well known in the chemistry of proteins.

The synthesis of peptides in homogeneous solution and in solid phase is well known.

In this respect, recourse may be had to the method of synthesis in homogeneous solution described by Houbenweyl in the work entitled "Methoden der Organischen Chemie" (Methods of Organic Chemistry) edited by E. WUNSCH., vol. 15-I and II, THIEME, Stuttgart 1974.

This method of synthesis consists of successively condensing either the successive aminoacids in twos, in the appropriate order or successive peptide fragments previously available or formed and containing already several aminoacyl residues in the appropriate order respectively. Except for the carboxyl and aminogroups which will be engaged in the formation of the peptide bonds, care must be taken to protect beforehand all other reactive groups borne by these aminoacyl groups or fragments. However, prior to the formation of the peptide bonds, the carboxyl groups are advantageously activated, according to methods well known in the synthesis of peptides. Alternatively, recourse may be had to coupling reactions bringing into play conventional coupling reagents, for instance of the carbodiimide type, such as 1-ethyl-3-(3-dimethyl-aminopropyl)-carbodiimide. When the aminoacid group used carries an additional amine group (e.g. lysine) or another acid function (e.g. glutamic acid), these groups may be protected by carbobenzoxy or t-butyloxycarbonyl groups, as regards the amine groups, or by t-butylester groups, as regards the carboxylic groups. Similar procedures are available for the protection of other reactive groups. For example, SH group (e.g. in cysteine) can be protected by an acetamidomethyl or paramethoxybenzyl group.

In the case of progressive synthesis, aminoacid by aminoacid, the synthesis starts preferably by the condensation of the C-terminal aminoacid with the aminoacid which corresponds to the neighboring aminoacyl group in the desired sequence and so on, step by step, up to the N-terminal aminoacid. Another preferred technique can be relied upon is that described by R.D. Merrifield in "solid phase peptide synthesis" (J. Am. Chem. Soc., 45, 2149-2154).

In accordance with the Merrifield process, the first C-terminal aminoacid of the chain is fixed to a suitable porous polymeric resin, by means of its carboxylic group, the amino group of said aminoacid then being protected, for example by a t-butyloxycarbonyl group.

When the first C-terminal aminoacid is thus fixed to the resin, the protective group of the amine group is removed by washing the resin with an acid, i.e. trifluoroacetic acid, when the protective group of the amine group is a t-butyloxycarbonyl group.

Then the carboxylic group of the second aminoacid which is to provide the second aminoacyl group of the desired peptide sequence, is coupled to the deprotected amine group of the C-terminal aminoacid fixed to the resin. Preferably, the carboxyl group of this second aminoacid has been activated, for example by dicyclohexylcarbodiimide, while its amine group has been protected, for example by a t-butyloxycarbonyl group. The first part of the desired peptide chain, which comprising the first two aminoacids, is thus obtained. As previously, the amine group is then deprotected, and one can further proceed with the fixing of the next aminoacyl group and so forth until the whole peptide sought is obtained.

The protective groups of the different side groups, if any, of the peptide chain so formed can then be removed. The peptide sought can then be detached from the resin, for example, by means of hydrofluoric acid, and finally recovered in pure form from the acid solution according to conventional procedures.

As regards the peptide sequences of smallest size and bearing an epitope or imunogenic determinant, and more particularly those which are readily accessible by chemical synthesis, it may be required, in order to increase their in vivo immunogenic character, to couple or "conjugate" them covalently to a physiologically acceptable and non toxic carrier molecule.

By way of examples of carrier molecules or macromolecular supports which can be used for making the conjugates according to the invention, will be mentioned natural proteins, such as tetanic toxoid, ovalbumin, serum-albumins, hemocyanins, etc.. Synthetic macromolecular carriers, for example polysines or poly (D-L-alanine)-poly(L-lysine)s, can be used too.

Other types of macromolecular carriers which can be used, which generally have molecular weights higher than 20,000, are known from the literature.

The conjugates can be synthesized by known processes, such as described by Frantz and Robertson in "Infect. and Immunity", 33, 193-198 (1981), or by P.E. Kauffman in Applied and Environmental Microbiology, October 1981, Vol. 42, n° 4, 611-614.

For instance the following coupling agents can be used : glutaric aldehyde, ethyl chloroformate, water-soluble carbodiimides (N-ethyl-N'(3-dimethylaminopropyl) carbodiimide, HCl), diisocyanates, bis-diazobenzidine, di- and trichloro-s-triazines, cyanogen bromides, benzoquinone, as well as coupling agents mentioned in "Scand. J. Immunol., 1978, vol. 8, p. 7-23 (Avrameas, Ternynck, Guesdon).

Any coupling process can be used for bonding one or several reactive groups of the peptide, on the one hand, and one or several reactive groups of the carrier, on the other hand. Again coupling is advantageously achieved between carboxyl and amine groups carried by the peptide and the carrier or vice-versa in the presence of a coupling agent of the type used in protein synthesis, i.e. 1-ethyl-3-(3-dimethylaminopropyl)-carbodiimide, N-hydroxybenzotriazole, etc.. Coupling between amine groups respectively borne by the peptide and the carrier can also be made with glutaraldehyde, for instance, according to the method described by BOQUET, P. et al. (1982) Molec. Immunol., 19, 1441-1549, when the carrier is hemocyanin.

The immunogenicity of epitope-bearing-peptides can also be reinforced, by oligomerisation thereof, for example in the presence of glutaraldehyde or any other suitable coupling agent. In particular, the invention relates to the water soluble immunogenic oligomers thus obtained, comprising particularly from 2 to 10 monomer units.

The glycoproteins, proteins and polypeptides (generally designated hereafter as "antigens" of this invention, whether obtained in a purified state from LAV virus preparations or - by recombinant DNA technology are useful in processes for the detection of the presence of anti-LAV antibodies in biological media, particularly biological fluids such as sera from man or animals, particularly with a view of possibly diagnosing LAS or AIDS.

Particularly the invention relates to an in vitro process of diagnosis making use of an envelope glycoprotein (or of a polypeptide bearing an epitope of this glycoprotein) for the detection of anti-LAV antibodies in the sera of persons who carry them. Other polypeptides - particular those carrying an epitope of a core protein - can be used too.

A preferred embodiment of the process of the invention comprises :

- depositing a predetermined amount of one or several of said antigens in the wells of a titration miroplate :
- introducing of increasing dilutions of the biological fluid, i.e. serum to be diagnosed into these wells :
- incubating the microplate :
- washing carefully the microplate with an appropriate buffer :
- adding to the wells specific labelled antibodies directed against blood immunoglobulins and
- detecting the antigen-antibody-complex formed, which is then indicative of the presence of LAV antibodies in the biological fluid.

Advantageously, the labelling of the anti-immunoglobulin antibodies is achieved by an enzyme selected fro among those which are capable of hydrolysing a substrate, which substrate undergoes a modification of its radiation-absorption, at least within a predetermined band of wavelength. The detection of the substrate, preferably comparatively with respect to a control, then provides a measurement of the potential risks or of the effective presence of the disease.

Thus preferred methods are immuno-enzymatic or also immunofluorescent detections, in particular according to the ELISA technique. Antibody titrations may be determinations by immunofluorescence or direct or indirect immunoenzymatic determinations. Quantitative titrations of antibodies in the sera studied can be made.

The invention also relates to the diagnostic kits, themselves, for the in vitro detection of antibodies against the LAV viruses, which kits comprise any of the polypeptides identified herein, and all the biological and to chemical reagents, as well as the equipment, necessary for peforming diagnostic assays. Preferred kits comprise all reagents required for carrying out ELISA assays. Thus preferred kits will include, in addition to any of said polypeptides, suitable buffers and anti-human immunoglobulins, which anti-human immunoglobulins are labelled either with an immunofluorescent molecule or by an enzyme. In the latter instance preferred kits then also comprise a substrate hydrolysable by the enzyme and providing a signal, particularly modified absorption of a radiation, at least in a determined wavelength, which signal is then indicative of the presence of antibody in the biological fluid to be assayed with said kit.

The invention also relates to vaccine compositions whose active principle is to be constituted by any of the antigens, i.e. the hereabove disclosed polypeptides whole antigens, fusion polypeptides or oligopeptides in association with a suitable pharmaceutical or physiologically acceptable carrier.

A first type of preferred active principle is the gp110 immungen.

Other preferred active principes to be considered in that field consist of the peptides containing less than 250 aminoacids, preferably less than 150, as deducible for the complete genomes of the LAV viruses, and even more preferably those peptides which contain one or more groups selected from Asn-X-Ser and Asn-X-Ser as defined above. By way of example having no limitative character, there may be mentioned that suitable dosages of the vaccine compositions are those which are effective to elicit antibodies in vivo, in the host, particularly a human host. Suitable doses range from 10 to 500 micrograms of polypeptide, protein or glycoprotein per kg, for instance 50 to 100 micrograms per kg.

The different peptides according to this invention can also be used themselves for the production of antibodies, preferably monoclonal antibodies specific for the different peptides respectively. For the production of hybridomas secreting said monoclonal antibodies, conventional production and screening methods are used. These monoclonal antibodies, which themselves are part of the invention then provide very useful tools for the identification and even determination of relative proportions of the different polypeptides or proteins in biological samples, particularly human samples containing LAV or related viruses.

The invention further relates to the hosts (prokaryotic or eukaryotic cells) which are transformed by the above mentioned recombinants and which are capable of expressing said DNA fragments.

Finally, the invention also concerns vectors for the transformation of eukaryotic cells of human origin, particularly lymphocytes, the polymerases of which are capable of recognizing the LTRs of LAV. Particularly said vectors are characterized by the presence of an LAV LTR therein, said LTR being then active as a promoter enabling the efficient transcription and translation in a suitable host of a DNA insert coding for a determined protein placed under its control.

Needless to say that the invention extends to all variants of genomes and corresponding DNA fragments (ORFs) having substantially equivalent properties, all of said genomes belonging to retroviruses which can be considered as equivalents of LAV.

It must be understood that the claims which follow are also intended to cover all equivalents of the products (glycoproteins, polypeptides, DNAs, etc..), whereby an equivalent is a product, i.e. a polypeptide which may distinguish from a determined one defined in any of said claims, say through one or several aminoacids, while still having substantially the same immunological or immunogenic properties. A similar rule of equivalency shall apply to the DNAs, it being understood that the rule of equivalency will then be tied to the rule of equivalency pertaining to the polypeptides which they encode.

It will also be understood that all the literature referred to hereinbefore or hereinafter, and all patent applications or patents, not specifically identified herein but which form counterparts of those specifically designated herein, must be considered as incorporated herein by reference.

REFERENCES

Alizon, M., Sonigo, P., Barré-Sinoussi, F., Chermann, J.C., Tiollais, P., Montagnier, L. and Wain-Hobson, S. (1984). Molecular cloning of lymphadenopathy-associated virus. Nature, in press.

Arya, S.K., Gallo, R.C., Hahn, B.H., Shaw, G.M., Popovic, M., Salahuddin, S.Z. and Wong-Staal, F. (1984). Homology of genome of AIDS-associated virus with genomes of human T-cell leukemia lymphoma viruses. Science 225, 927-930.

Barré-Sinoussi, F., Chermann, J.C., Rey, F., Nugeybe, M.T., Chamaret, S., Gruest, J., Dauguet, C., Axler-Blin, C., Vézinet-Brun F., Rouzioux, C., Rozenbaum, W. and Montagnier, L. (1983). Isolation of a T-lymphotropic retrovirus from a patient at risk of Acquired Immune Deficiency Syndrome(AIDS). Science 220, 868-870.

Biggen, M.D., Gibson, T.J. and Hong, G.F. (1983). Buffer gradient gels and $^{35}$S label as an aid to rapid DNA sequence determination. Proc. Natl. Acad. Sci. USA 80, 3963-3965.

Bird, A.P. (1980). DNA methylation and the frequency of CpG in animal DNA. Nucl. Acids Res. 8, 1499-1504.

Brun-Vézinet, F., Rouzioux, C., Barré-Sinoussi, F., Klatzmann, D., Saimot, A.G., Rozembaum, W., Montagnier, L. and Chermann, J.C. (1984). Detection of IgG antibodies to lymphadenopathy associated virus (LAV) by ELISA, in patients with acquired immuno-deficiency syndrome of lymphadenopathy syndrome. Lancet I, 1253-1256.

Chen, H.R. and Barker, W.C. (1984). Nucleotide sequences of the retroviral long terminal repeats and their adjacent regions. Nucl. Acids Res. 12, 1767-1778.

Chen, I.S.Y., Mc Laughlin, J., Gasson, J.C., Clark, S.C. and Golde, D.W. (1983). Molecular characterization of genome of a novel human T-cell leukaemia virus. Nature 305, 502-505.

Chiu, I.M., Callahan, R., Tronick, S.R., Scholm, J. and Aaronson, S.A. (1984). Major pol gene progenitors in the evolution of oncornaviruses. Science 223, 364-370.

Cianciolo, G.J., Kipnis, R.J. and Snyderman, R. (1984). Similarity between p15E of murine and feline viruses and p2l of HTLV. Nature 311, 515.

Daly, H.M. and Scott, G.L. (1983). Fatal AIDS in a haemophiliae in the U.K. Lancet II, 1190.

Dittmar, K.J. and Moelling, K. (1978). Biochemical properties of p15-associated protease in an avion RNA tumor virus. J. Virol. 28, 106-118.

Donehower, L.A., Huang, A.L. and Hager, G.L. (1981). Regulatory and coding potential of the mouse mammary tumour virus long terminal redundancy. J. Virol. 37, 226-238.

Gottlieb, M.S., Schroff, R., Schanler, H.M., Weisman, J.D., Fan P.T., Wolf R.A., Saxon, A. (1981). Pneumocytis carinii pneumonia and mucosal candidiasis in previously healthy homosexual men : Evidence of a new acquired cellular immuno-deficiency. N. Engl. J. Med. 305, 1426-1431.

Hahn, B.H., Shaw, G.M., Arya, S.U., Popovic, M., Gallo, R.C. and Wong- Stall, F. (1984). Molecular cloning and characterization of the HTLV-III virus associated with AIDS. Nature 312, 166-169.

Harris, J.D., Scott, J.V., Taylor, B., Brahic, M., Stowring, L., Ventura, P., Haase, A.T. and Peluso, R. (1981). Visna virus DNA : discovery of a novel gapped structure. Virology 113, 573-583.

Kiyokawa, T., Yoshikura, H., Hattori, S., Secki, M. and Yoshida, M. (1984). Envelope proteins of human T-cell leukemia virus : expression in Escherischia coli and its application to studies of env gene functions. Proc. Natl. Acad. Sci. USA 81, 6202-6206.

Klatzmann, D., Barré-Sinoussi, F., Nugeyre, M.T., Dauguet, C., Vilmer, E., Griscelli, C., Brun-Vézinet, F., Rouzioux, C., Gluckman, J.C., Chermann, J.C. and Montagnier, L. (1984). Selective tropism of lymphadenopathy associated virus (LAV) for helper-inducer T-lymphocytes. Sciences 225, 59-63.

Kozak, M. (1984). Compilation and analysis of sequences upstream from the transcriptional start site in eucaryotic mRNAs. Nucl. Acids Res. 12, 857-872.

Levy, J.A., Hoffman, A.D., Kramer, S.M., Lanois, J.A., Shimabukuro, J.M. and Oskiro, L.S. (1984). Isolation of lymphocytopathic retroviruses from San Francisco patients with AIDS. Science 225, 840-842.

Masur, H., Michelis, M.A., Greene, J.B., Onovato, I., Van de Stowe, R.A., Holzman, R.S., Wormser, G., Brettman, L., Lange, M., Murray, H.W., Cunningham-Rundles, S. (1981). An outbreak of community-acquired pneumocystis carinii pneumonia: Initial manifestation of cellular immune dysfunction. N. Engl. J. Med. 305, 1431-1438.

Misra, T.K., Grandgenett, D.P. and Parsons, J.T. (1982). Avian retrovirus pp32 DNA-binding protein. I. Recognition fo specific sequences on retrovirus DNA terminal repeats. J. Virol. 44, 330-343.

Montagnier, L., et al., (1984). A new human T-lymphotropic retrovirus : characterization and possible role in lymphadenopathy and acquired immune deficiency syndromes. In human T-cell leukemia/lymphoma viruses. R.C. Gallo, M. Essex and L. Gross, eds. (Cold Spring Laboratory, New-York), pp 363-370.

Oroszlan, S., Copeland, T.D., Kalyanaraman, V.S., Sarngadharan, M.G., Schultz, A.M. and Gallo, R.C. (1984). Chemical analysis of human T-cell leukemia virus structural proteins. In HTLVS (R.C. Gallo, M.E. Essex and L. Gross, eds) Cold Spring Laboratory, New-York, pp 101-110.

Piot, P., Quinn, T.C., Taelmann, H., Feinsod, F.M. et al., (1984). Acquired immunodeficiency syndrome in a heterosexual population in Zaire. Lancet II, 65-69.

Popovic, M., Sarngadharan, M.G., Read, E. and Gallo, R.C. (1984). Detection, isolation, and continuous production of cytopathic retroviruses (HTLV-III) from patients with AIDS and pre-AIDS. Science 224, 497-500.

Querat, G., Barban, N., Sauze, N., Filippi, P., Vigne, R., Russo, P. and Vitu, C. (1984). Highly lytic and persistent lentiviruses naturally present in sheep with progressive pneumonia are genetically distinct. J. Virol. 52, 672-679.

Raba, M., Limburg, K., Burghagen, M., Katze, J.R., Simsek, M., Heckman, J.E., Rajbhandary, U.L., and Gross, H.J. (1979). Nucleotide sequence fo three isoaccepting lysine tRNAs from rabbit liver and SV40-transformed mouse fibroblasts. Eur. J. Biochem. 97, 305-318.

Rice, N.R., Stephens, R.M., Couez, D., Deschamps, J., Kettmann, R., Burny, A., and Gilden, R.V. (1984). The nucleotide sequence of the env gene and post-env region of bovine leukemia virus. Virology 138, 82-93.

Sagata, N., Yasunaga, T., Ogawa, Y., Tsuzuku-Kawamura, J. and Ikawa, Y. (1984). Bovine leukemia virus : Unique structural features of its long terminal repeats and its evolutionary relationship to human T-cell leukemia virus. Proc. Natl. Acad. Sci. USA 81, 4741-4745.

Sanger, F., Nicklen, S. and Coulsen, A.R. (1977). DNA sequencing with chain terminating inhibitors. Proc. Natl. Acad. Sci. USA 74, 5463-5467.

Schwartz, D.E., Tizard, R. and Gilbert, W. (1983). Nucleotide sequence of Rous sarcoma virus. Cell 32, 853-869.

Schüpbach, J., Popovic, M., Gilden, R.V., Gonda, M.A., Sarngadharan, M.G. and Gallo, R.C. (1984). Serological analysis of a subgroup of human T-lymphotropic retroviruses (HTLV-III) associated with AIDS. Science 224, 503-505.

Seiki, M., Hattori, S., Hirayama, Y. and Yoshida, M. (1983). Human adult T-cell leukemia virus : complete nucleotide sequence of the provirus genome integrated in leukemia cell DNA. Proc. Natl, Acad. Sci. USA 80, 3618-3622.

Shaw, G.M., Hahn, B.H., Arya, S.K., Groopman, J.E., Gallo, R.C., and Wong-Staal, F. (1984). Molecular characterization of human T-cell leukemia lymphotropic) virus type III in the Acquired Immune Deficiency Syndrome. Science 226, 1165-1171.

Shimotohno, k., and Temin, H.M. (1982). Spontaneous variation and synthesis in the U3 region of the long terminal repeat of avion retroviruses. J. Virol. 41, 1636171.

Shimotohno, K., Golde, D.M., Miwa, M., Sugimura, T. and Chen, I.S.Y. (1984). Nucleotide sequence analysis fo the long terminal repeat of human T-cell leukemia virus type II. Proc. Natl. Acad. Sci. USA 81, 1079-1083.

Shinnick, T.M., Lerner, R.A. and Sutcliffe, J.G. (1981). Nucleotide sequence of Moloney murine leukemia viruse. Nature 293, 543-548.

Srinivasan, A., Reddy, E.P., Dunn, C.Y. and Aaronson, S.A. (1984). Molecular dissection of transcriptional control elements with the long terminal repeat of retrovirus. Science 223, 286-289.

## Claims

**Claims for the following Contracting States : BE, CH, DE, FR, GB, IT, LI, LU, NL, SE**

1. Purified F antigen which corresponds to the polypeptide encoded by the nucleotide sequence comprised between nucleotides 8324 and 8971 of the LAV virus genome, of figure 4 or an antigen which has the same polypeptide backbone, having 206 aminoacids and whose amino-acid sequence is as follows:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

2. Polypeptide fragment comprised in the F antigen according to claim 1 characterized in that it is a fragment bearing an epitope characteristic of the antigen according to claim 1.

3. Polypeptide fragment according to claim 2, characterized in that it bears an immunogenic determinant.

4. Polypeptide fragment according to claim 3, characterized in that it is covalently coupled to a physiologically acceptable and non toxic carrier molecule in order to enhance its in vivo immunogenic character.

5. Polypeptide fragment according to claim 3, characterized in that it is under an oligomerised form.

6. Purified DNA capable of coding for the F antigen of claim 1 or a fragment according to anyone of claims 2 to 3.

7. The DNA of claim 6 which has the nucleotide structure of the fragment extending from positions 8324 to 8971 of the nucleotide sequence defined on figure 4.

8. Recombinant vector characterised in that it comprises a DNA sequence according to claim 6 or to claim 7.

9. Microorganism which is transformed by a vector according to claim 8 in conditions authorizing the expression in said microorganism of the DNA sequence included in the vector and coding for said F antigen.

10. Microorganism according to claim 9, characterised in that it is chosen among eukaryotic cells, particularly mammal cells or yeasts.

11. Process for the chemical synthesis of a peptide or polypeptide according to claim 1 or a fragment according to anyone of claims 2 or 3, characterised by:
    - successively condensing either the successive aminoacids in twos, in the appropriate order or successive peptide fragments previously available or formed and containing already several aminoacyl residues in the appropriate order respectively, all the reactive groups other than the carboxyl and aminogroups which will be engaged in the formation of the peptide bond being protected,
    - possibly activating the carboxylgroups prior to the formation of the peptide bond,
    - recovering the peptide or polypeptide formed.

12. Process for the preparation of the antigen according to claim 1 characterised by :
    - the transformation of prokaryotic or eukaryotic host with a suitable vector previously modified by a DNA according to claim 8 in conditions enabling the production of the desired peptide,
    - recovering and purifying the antigen thus obtained for instance by electrophoresis.

13. A process for the detection of the presence of anti-LAV antibodies in a biological medium which comprises contacting said biological medium with the polypeptide of anyone of claims 1 to 5 under conditions suitable for enabling a complex between said antibodies and said polypeptide to be formed, and detecting said complex as indicative of the presence of said antibodies in said biological fluid.

14. Vaccine composition characterized in that the active principle is constituted by the F antigen according to anyone of claims 1 to 5 in association with a suitable pharmaceutical or physiologically acceptable carrier.

15. Process for the production of monoclonal antibodies against F antigen according to claim 1 or against a polypeptide fragment according to anyone of claims 2 to 5, comprising the step of :
    - fusing myeloma cells with splenocytes of an animal previously immunised with the F antigen, to obtain hybridomas,
    - selecting the hybridomas producing antibodies having specificity for the F antigen,
    - recovering the antibodies against the F antigen.

16. Antibody characterized in that it is recognized by the F antigen according to claim 1 or by a polypeptide fragment according to anyone of claims 2 to 5.

17. Antibody according to claim 16, characterized in that it is a monoclonal antibody.

18. Use of antibodies according to claims 16 or 17, for the identification of the F antigen or even for the determination of relative proportion of this antigen, in a biological sample containing LAV or related viruses.

**Claims for the following Contracting State : AT**

1. Process for the chemical synthesis of a purified F antigen which corresponds to the polypeptide encoded by the nucleotidec sequence comprised between nucleotides 8324 and 8971 of the LAV virus genome, of figure 4 or an antigen which has the same polypeptide backbone, having 206 aminoacids and whose amino-acid sequence is as follows:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

or an immunogenic polypeptide fragment thereof, characterised by:
   - successively condensing either the successive aminoacids in twos, in the appropriate order or successive peptide fragments previously available or formed and containing already several aminoacyl residues in the appropriate order respectively, all the reactive groups other than the carboxyl and aminogroups which will be engaged in the formation of the peptide bond being protected,
   - possibly activating the carboxylgroups prior to the formation of the peptide bond,
   - recovering the antigen or its immunogenic fragment formed.

2. Process according to claim 1, characterized in that the synthesized polypeptide fragment bears an epitope characteristic of the F antigen.

3. Process according to claim 2, characterized in that the synthesized polypeptide fragment bears an immunogenic determinant.

4. Process for the preparation of an immunogenic polypeptide fragment having _in vivo_ increased immunogenic character, characterized in that the synthesized polypeptide fragment according to anyone of claims 1 to 3 is covalently coupled to a physiologically acceptable and non toxic carrier molecule.

5. Process for the preparation of an immunogenic polypeptide fragment having _in vivo_ increased immunogenic character, characterized in that the synthesized polypeptide fragment according to anyone of claims 1 to 3 is oligomerized in the presence of a suitable coupling agent.

6. Process for the preparation of the purified F antigen which corresponds to the polypeptide encoded by the nucleotidec sequence comprised between nucleotides 8324 and 8971 of the LAV virus genome, of figure 4 or an antigen which has the same polypeptide backbone, having 206 aminoacids and whose amino-acid sequence is as follows:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
```

```
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys
```

or an immunogenic polypeptide fragment thereof characterized by :
- the transformation of prokaryotic or eukaryotic host with a suitable vector previously modified by a DNA sequence extending from positions 8324 to 8971 of the nucleotidec sequence defined on figure 4, or a nucleotidec fragment thereof, in conditions enabling the production of the desired antigen,
- recovering and purifying the antigen thus obtained for instance by electrophoresis.

7. Process for the preparation of purified DNA coding for the F antigen, or a fragment of the F antigen, characterized by :
- cleaving the entire DNA corresponding to the complete genome of an LAV species by digestion with a suitable restriction enzyme,
- recovering the relevant fragments coding for the F antigen or a fragment thereof.

8. Process according to claim 7, characterized in that the recovered DNA has the nucleotide structure of the fragment extending from positions 8324 to 8971 of the nucleotidec sequence defined in figure 4.

9. Process for the preparation of a recombinant vector, characterised in that a vector is modified with a DNA sequence obtained by a process according to claim 7 or to claim 8.

10. Process for the preparation of a recombinant microorganism, characterized by transforming a microorganism wih a vector as obtained by a process according to claim 9, in conditions authorizing the expression in said microorganism of the DNA sequence included in the vector and coding for said F antigen or an immunogenic fragment thereof.

11. Process according to claim 10, characterized in that eukaryotic cells, particularly mammal cells or yeasts are transformed.

12. A process for the detection of the presence of anti-LAV antibodies in a biological medium which comprises contacting said biological medium with the polypeptide of anyone of claims 1 to 5 under conditions suitable for enabling a complex between said antibodies and said polypeptide to be formed, and detecting said complex as indicative of the presence of said antibodies in said biological fluid.

13. Process for the preparation of a vaccine composition containing as active principle the F antigen or an immunogenic fragment thereof as obtained by a process according to anyone of claims 1 to 5, characterized in that it is associated with a suitable pharmaceutical or a physiologically acceptable carrier.

14. Process for the production of monoclonal antibodies against F antigen as obtained by a process according to claim 1 or against an immunogenic polypeptide fragment thereof, comprising the step of :
- fusing myeloma cells with ophenocytes of an animal previously immunised with the F antigen or an immunogenic polypeptide fragment thereof, to obtain hybridomas,

- selecting the hybridomas producing antibodies having specificity for the F antigen, or an immunogenic polypeptide fragment thereof,
- recovering the antibodies recognized by the F antigen or an immunogenic polypeptide fragment thereof.

15. Use of antibodies according to claims 12 or 14, for the identification of the F antigen or even for the determination of relative proportion of this antigen, in a biological sample containing LAV or related viruses.

**Patentansprüche**

**Patentansprüche für folgende Vertragsstaaten : BE, CH, DE, FR, GB, IT, LI, LU, NL, SE**

1. Gereinigtes F-Antigen dem Polypeptid entsprechend, das von der Nucleotidsequenz codiert wird, die die Nucleotide 8324 bis 8971 des LAV-Virusgenoms von Figur 4 umfaBt, oder ein Antigen mit dem gleichen Polypeptidgrundgerüst, das 206 Aminosäuren besitzt und dessen Aminosäuresequenz wie folgt ist:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

2. Polypeptidfragment, das in dem F-Antigen nach Anspruch 1 enthalten ist, dadurch gekennzeichnet, daß es ein Fragment ist, das ein für das Antigen nach Anspruch 1 charakteristisches Epitop trägt.

3. Polypeptidfragment nach Anspruch 2, dadurch gekennzeichnet, daß es eine immunogene Determinante trägt.

4. Polypeptidfragment nach Anspruch 3, dadurch gekennzeichnet, daß es mit einem physiologisch verträglichen und nicht-toxischen Trägermolekül zur Steigerung seiner immunogenen Eigenschaft in vivo kovalent verknüpft ist.

5. Polypeptidfragment nach Anspruch 3, dadurch gekennzeichnet, daß es in einer oligomerisierten Form vorliegt.

6. Gereinigte DNA, die zur Codierung des F-Antigens nach Anspruch 1 oder eines Fragments nach einem der Ansprüche 2 bis 3 befähigt ist.

7. DNA nach Anspruch 6, die die Nucleotidstruktur des Fragments besitzt, das sich von Position 8324 bis 8971 der in Figur 4 definierten Nucleotidsequenz erstreckt.

8. Rekombinanter Vektor, dadurch gekennzeichnet, daß er eine DNA-Sequenz nach Anspruch 6 oder 7 umfaßt.

9. Mikroorganismus, der mit einem Vektor nach Anspruch 8 unter Bedingungen transformiert wurde, die die Expression der von dem Vektor eingeschlossenen und das F-Antigen codierenden DNA-Sequenz in dem Mikroorganismus erlauben.

10. Mikroorganismus nach Anspruch 9, dadurch gekennzeichnet, daß er aus eukaryontischen Zellen, bevorzugt Säugerzellen oder Hefen, ausgewählt wird.

11. Verfahren zur chemischen Synthese eines Peptids oder eines Polypeptids nach Anspruch 1 oder eines Fragments nach einem der Ansprüche 2 oder 3, dadurch gekennzeichnet, daß:
    die aufeinanderfolgenden Aminosäuren in Zweiergruppen in der richtigen Reihenfolge bzw. aufeinanderfolgende, bereits vorhandene oder gebildete Peptidfragmente, die bereits verschiedene Aminoacylreste in der richtigen Reihenfolge enthalten, schrittweise kondensiert werden, wobei alle reaktiven Gruppen, außer den an der Bildung der Peptidbindung beteiligten Carboxyl- und Aminogruppen, geschützt sind,
    möglicherweise die Aktivierung der Carboxylgruppen vor der Bildung der Peptidbindung,
    Gewinnung des gebildeten Peptids oder Polypeptids.

12. Verfahren zur Herstellung des Antigens nach Anspruch 1, dadurch gekennzeichnet, daß
    ein prokaryontischer oder eukaryontischer Wirt mit einem geeigneten Vektor, der zuvor durch eine DNA nach Anspruch 8 modifiziert wurde, unter Bedingungen transformiert wird, die die Herstellung des gewünschten Peptids erlauben,
    Gewinnung und Reinigung des so erhaltenen Antigens, z.B. durch Elektrophorese.

13. Verfahren zum Nachweis der Anwesenheit von anti-LAV-Antikörpern in einem biologischen Medium, umfassend das Inkontaktbringen des biologischen Mediums mit dem Polypeptid nach einem der Ansprüche 1 bis 5 unter Bedingungen, die geeignet sind, um die Bildung eines Komplexes zwischen den Antikörpern und dem Polypeptid zu ermöglichen, und Nachweis dieses Komplexes als Hinweis auf das Vorhandensein der Antikörper in der biologischen Flüssigkeit.

14. Impfstoffzusammensetzung, dadurch gekennzeichnet, daß, das F-Antigen nach einem der Ansprüche 1 bis 5 in Verbindung mit einem geeigneten pharmazeutischen oder physiologisch verträglichen Träger den aktiven Grundbestandteil ausmacht.

15. Verfahren zur Herstellung von monoclonalen Antikörpern gegen das F-Antigen nach Anspruch 1 oder gegen ein Polypeptidfragment nach einem der Ansprüche 2 bis 5, umfassend die Schritte:
    Fusionieren von Myelomzellen mit Splenocyten eines zuvor mit dem F-Antigen immunisierten Tieres zum Erhalt von Hybridomen,
    Auswählen der Hybridome, die Antikörper mit Spezifität für das F-Antigen herstellen,
    Gewinnung der Antikörper gegen das F-Antigen.

16. Antikörper, dadurch gekennzeichnet, daß er von dem F-Antigen nach Anspruch 1 oder einem Polypeptid nach einem der Ansprüche 2 bis 5 erkannt wird.

17. Antikörper nach Anspruch 16, dadurch gekennzeichnet, daß es ein monoclonaler Antikörper ist.

18. Verwendung der Antikörper nach Anspruch 16 oder 17 zum Nachweis des F-Antigens oder auch zur Bestimmung des relativen Anteils dieses Antigens in einer LAV- oder verwandte Viren enthaltenden biologischen Probe.

**Patentansprüche für folgenden Vertragsstaat : AT**

1. Verfahren zur chemischen Synthese eines gereinigten F-Antigens dem Polypeptid entsprechend, das von der Nucleotidsequenz codiert wird, die die Nucleotide 8324 bis 8971 des LAV-Virusgenoms von Figur 4 umfaßt, oder eines Antigens mit dem gleichen Polypeptidgrundgerüst, das 206 Aminosäuren besitzt und dessen Aminosäuresequenz wie folgt ist:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

oder eines immunogenen Polypeptidfragments davon, dadurch gekennzeichnet, daß

die aufeinanderfolgenden Aminosäuren in Zweiergruppen in der richtigen Reihenfolge bzw. aufeinanderfolgende, bereits vorhandene oder gebildete Peptidfragmente, die bereits verschiedene Aminoacylreste in der richtigen Reihenfolge enthalten, schrittweise kondensiert werden, wobei alle reaktiven Gruppen, außer den an der Bildung der Peptidbindung beteiligten Carboxyl- und Aminogruppen, geschützt sind,

möglicherweise die Aktivierung der Carboxylgruppen vor der Bildung der Peptidbindung,
Gewinnung des Antigens oder des davon gebildeten immunogenen Fragments.

2. Verfahren nach Anspruch 1, dadurch gekennzeichnet, daß das synthetisierte Polypeptidfragment ein für das F-Antigen charakteristisches Epitop trägt.

3. Verfahren nach Anspruch 2, dadurch gekennzeichnet, daß das synthetisierte Polypeptidfragment eine immunogene Determinante trägt.

4. Verfahren zur Herstellung eines immunogenen Polypeptidfragments mit einer gesteigerten immunogenen Eigenschaft in vivo, dadurch gekennzeichnet, daß das synthetisierte Polypeptidfragment nach einem der Ansprüche 1 bis 3 mit einem physiologisch verträglichen und nicht-toxischen Trägermolekül kovalent verknüpft ist.

5. Verfahren zur Herstellung eines immunogenen Polypeptidfragments mit einer gesteigerten immunogenen Eigenschaft in vivo, dadurch gekennzeichnet, daß das synthetisierte Polypeptidfragment nach einem der Ansprüche 1 bis 3 in Anwesenheit eines geeigneten Kopplungsmittels in einer oligomerisierten Form vorliegt.

6. Verfahren zur Herstellung eines gereinigten F-Antigens dem Polypeptid entsprechend, das von der Nucleotidsequenz codiert wird, die die Nucleotide 8324 bis 8971 des LAV-Virus-Genoms von Fig. 4 umfaßt, oder eines Antigens mit dem gleichen Polypeptidgrundgerüst, das 206 Aminosäuren besitzt und dessen Aminosäuresequenz wie folgt ist:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys
```

oder eines immunogenen Polypeptidfragments davon, dadurch gekennzeichnet, daß

ein prokaryontischer oder eukaryontischer Wirt mit einem geeigneten Vektor, der zuvor mit einer sich von der Position 8324 bis 8971 der in Fig. 4 definierten Nucleotidsequenz erstreckenden DNA-Sequenz oder eines Nucleotidfragments davon modifiziert wurde, unter Bedingungen transformiert wird, die die Herstellung des gewünschten Peptids erlauben,

Gewinnung und Reinigung des so erhaltenen Antigens, z.B. durch Elektrophorese.

7. Verfahren zur Herstellung gereinigter, das F-Antigen oder ein Fragment des F-Antigens codierender DNA, dadurch gekennzeichnet, daß

die gesamte dem Gesamtgenom einer LAV-Art entsprechende DNA durch Spaltung mit einem geeigneten Restriktionsenzym gespalten wird,

die relevanten, das F-Antigen oder ein Fragment davon codierenden Fragmente gewonnen werden.

8. Verfahren nach Anspruch 7, dadurch gekennzeichnet, daß die gewonnene DNA die Nucleotidstruktur des Fragments besitzt, das sich von Position 8324 bis 8971 der in Fig. 4 definierten Nucleotidsequenz erstreckt.

9. Verfahren zur Herstellung eines rekombinanten Vektors, dadurch gekennzeichnet, daß ein Vektor mit einer nach einem Verfahren nach Anspruch 7 oder 8 erhaltenen DNA-Sequenz modifiziert wird.

10. Verfahren zur Herstellung eines rekombinanten Mikroorganismus, dadurch gekennzeichnet, daß ein Mikroorganismus mit einem durch ein Verfahren nach Anspruch 9 erhaltenen Vektor unter Bedingungen transformiert wird, die die Expression der von dem Vektor eingeschlossenen und das F-Antigen oder ein immunogenes Fragment davon codierenden DNA-Sequenz in dem Mikroorganismus erlauben.

11. Verfahren nach Anspruch 10, dadurch gekennzeichnet, daß eukaryontische Zellen, bevorzugt Säugerzellen oder Hefen, transformiert werden.

12. Verfahren zum Nachweis der Anwesenheit von anti-LAV-Antikörpern in einem biologischen Medium, umfassend das Inkontaktbringen des biologischen Mediums mit dem Polypeptid nach einem der Ansprüche 1 bis 5, unter Bedingungen, die geeignet sind, um die Bildung eines komplexes zwischen den Antikörpern und dem Polypeptid zu ermöglichen, und Nachweis dieses komplexes als Hinweis auf das Vorhandensein der Antikörper in der biologischen Flüssigkeit.

13. Verfahren zur Herstellung einer Impfstoffzusammensetzung, die als aktiven Grundbestandteil das durch ein Verfahren nach einem der Ansprüche 1 bis 5 erhaltene F-Antigen oder ein immunogenes Fragment davon enthält, dadurch gekennzeichnet, daß es mit einem geeigneten pharmazeutischen oder physiolo-

24

gisch verträglichen Träger vereinigt wird.

14. Verfahren zur Herstellung von monoclonalen Antikörpern gegen das durch ein Verfahren nach Anspruch 1 erhaltene F-Antigen oder gegen ein immunogenes Polypeptidfragment davon, umfassend die Schritte:

Fusionieren von Myelomzellen mit Splenocyten eines zuvor mit dem F-Antigen oder einem immunogenen Polypeptidfragment davon immunisierten Tieres zum Erhalt von Hybridomen,

Auswählen der Hybridome, die Antikörper mit Spezifität für das F-Antigen oder ein immunogenes Polypeptidfragment davon herstellen,

Gewinnung der von dem F-Antigen oder einem immunogenen Polypeptidfragment davon erkannten Antikörper.

15. Verwendung der Antikörper nach Anspruch 12 oder 14 zum Nachweis des F-Antigens oder auch zur Bestimmung des relativen Anteils dieses Antigens in einer LAV- oder verwandte Viren enthaltenden biologischen Probe.

## Revendications

**Revendications pour les Etats contractants suivants : BE, CH, DE, FR, GB, IT, LI, LU, NL, SE**

1. Antigène F purifié qui correspond au polypeptide codé par la séquence de nucléotides comprise entre les nucléotides 8324 et 8971 du génome du virus LAV, de la figure 4 ou un antigène ayant la même ossature polypeptidique, ayant 206 acides aminés et dont la séquence en acides aminés est la suivante:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

2. Fragment polypeptidique compris dans l'antigène F selon la revendication 1, caractérisé en ce qu'il s'agit d'un fragment portant un épitope caractéristique de l'antigène selon la revendication 1.

3. Fragment polypeptidique selon la revendication 2, caractérisé en ce qu'il porte un déterminant immunogène.

4. Fragment polypeptidique selon la revendication 3, caractérisé en ce qu'il est couplé de façon covalente à une molécule porteuse physiologiquement acceptable et non toxique, de façon à augmenter son caractère immunogène in vivo.

5. Fragment polypeptidique selon la revendication 3, caractérisé en ce qu'il est sous une forme oligomérisée.

6. ADN purifié capable de coder pour l'antigène F selon la revendication 1 ou un fragment selon l'une quelconque des revendications 2 à 3.

7. ADN selon la revendication 6 qui a la structure nucléotidique du fragment s'étendant entre les positions 8324 et 8971 de la séquence nucléotidique définie à la figure 4.

8. Vecteur recombinant caractérisé en ce qu'il comprend une séquence d'ADN selon la revendication 6 ou la revendication 7.

9. Microorganisme transformé par un vecteur selon la revendication 8, dans des conditions permettant l'expression dans ce microorganime de la séquence d'ADN incluse dans le vecteur et codant pour le dit antigène F.

10. Microorganisme selon la revendication 9, caractérisé en ce qu'il est choisi parmi les cellules eucaryotes, en particulier les cellules de mammifère ou les levures.

11. Procédé pour la synthèse chimique d'un peptide ou d'un polypeptide selon la revendication 1 ou d'un fragment selon l'une quelconque des revendications 2 ou 3, caractérisé par:
    - la condensation successive soit d'acides aminés successifs deux par deux, selon l'ordre approprié, soit de fragments polypeptidiques successifs préalablement disponibles ou formés et contenant déjà plusieurs résidus aminoacyle dans l'ordre approprié respectivement, tous les groupes réactifs autres que le carboxyle et les groupes aminés qui seront engagés dans la formation de la liaison peptidique étant protégée,
    - éventuellement l'activation des groupes carboxyles avant la formation de la liaison peptidique,
    - la récupération du peptide ou du polypeptide formé.

12. Procédé pour la préparation d'un antigène selon la revendication 1 caractérisé par:
    - la transformation d'un hôte procaryote ou eucaryote avec un vecteur approprié préalablement modifié par un ADN selon la revendication 8 dans des conditions permettant la production du peptide désiré,
    - la récupération et la purification de l'antigène ainsi obtenu par exemple par électrophorèse.

13. Procédé pour la détection de la présence d'anticorps anti-LAV dans un milieu biologique, qui comprend la mise en contact du dit milieu biologique avec le polypeptide selon l'une quelconque des revendications 1 à 5 dans des conditions appropriées pour permettre la formation d'un complexe entre les dits anticorps et le dit polypeptide, et la détection du dit complexe comme indication de la présence des dits anticorps dans le dit fluide biologique.

14. Composition de vaccin caractérisée en ce que le principe actif est constitué par l'antigène F selon l'une quelconque des revendications 1 à 5, en association avec un véhicule pharmaceutiquement acceptable ou physiologiquement acceptable.

15. Procédé pour la production d'anticorps monoclonaux contre l'antigène F selon la revendication 1 ou contre un fragment polypeptidique selon une quelconque des revendications 2 à 5 comprenant les étapes de:
    - fusion de cellules de myélome avec des splénocytes d'un animal préalablement immunisé avec l'antigène F, pour obtenir des hybridomes,
    - sélection des hybridomes produisant les anticorps ayant la spécificité pour l'antigène F,
    - récupération des anticorps contre l'antigène F.

16. Anticorps caractérisé en ce qu'il est reconnu par l'antigène F selon la revendication 1 ou par un fragment polypeptique selon l'une quelconque des revendications 2 à 5.

17. Anticorps selon la revendication 16, caractérisé en ce qu'il s'agit d'un anticorps monoclonal.

18. Utilisation d'anticorps selon les revendications 16 ou 17, pour l'identification de l'antigène F ou même pour la détermination de la proportion relative de cet antigène, dans un échantillon biologique contenant LAV ou des virus apparentés.

**Revendications pour l'Etat contractant suivant : AT**

1. Procédé pour la synthèse chimique d'un antigène F purifié qui correspond au polypeptide codé par la séquence de nucléotides comprise entre les nucléotides 8324 et 8971 du génome du virus LAV, de la figure 4 ou un antigène ayant la même ossature polypeptidique, ayant 206 acides aminés et dont la séquence en acides aminés est la suivante:

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys.
```

ou un fragment polypeptidique immunogène de cet antigène, caractérisé par:
- la condensation successive soit d'acides aminés successifs deux par deux, selon l'ordre approprié, soit de fragments polypeptidiques successifs préalablement disponibles ou formés et contenant déjà plusieurs résidus aminoacyle dans l'ordre approprié respectivement, tous les groupes réactifs autres que le carboxyle et les groupes aminés qui seront engagés dans la formation de la liaison peptidique étant protégée,
- éventuellement l'activation des groupes carboxyles avant la formation de la liaison peptidique,
- la récupération de l'antigène ou de son fragment immunogène formé.

2. Procédé selon la revendication 1, caractérisé en ce que le fragment polypeptidique synthétisé porte un épitope caractéristique de l'antigène F.

3. Procédé selon la revendication 2, caractérisé en ce que le fragment polypeptidique synthétisé porte un déterminant immunogène.

4. Procédé pour la préparation d'un fragment polypeptidique immunogène dont le caractère immunogène est augmenté in vivo, caractérisé en ce que le fragment polypeptidique synthétisé selon l'une quelconque des revendications 1 à 3 est couplé de façon covalente à une molécule porteuse physiologiquement acceptable et non toxique.

5. Procédé pour la préparation d'un fragment polypeptidique immunogène dont le caractère immunogène est augmenté in vivo, caractérisé en ce que le fragment polypeptidique synthétisé selon l'une quelconque des revendications 1 à 3 est sous une forme oligomérisée en présence d'un agent de couplage approprié.

6. Procédé pour la préparation d'un antigène F purifié qui correspond au polypeptide codé par la séquence de nucléotides comprise entre les nucléotides 8324 et 8971 du génome du virus LAV, de la figure 4 ou un antigène qui a la même ossature polypeptidique, ayant 206 acides aminés et dont la séquence d'acides aminés est la suivante :

```
AspArgAlaTrpLysGlyPheCysTyrLysMetGlyGlyLysTrpSerLysSer
SerValValGlyTrpProThrValArgGluArgMetArgArgAlaGluProAla
AlaAspGlyValGlyAlaAlaSerArgAspLeuGluLysHisGlyAlaIleThr
SerSerAsnThrAlaAlaThrAsnAlaAlaCysAlaTrpLeuGluAlaGlnGlu
GluGluGluValGlyPheProValThrProGlnValProLeuArgProMetThr
TyrLysAlaAlaValAspLeuSerHisPheLeuLysGluLysGlyGlyLeuGlu
GlyLeuIleHisSerGlnArgArgGlnAspIleLeuAspLeuTrpIleTyrHis
```

```
ThrGlnGlyTyrPheProAspTrpGlnAsnTyrThrProGlyProGlyValArg
TyrProLeuThrPheGlyTrpCysTyrLysLeuValProValGluProAspLys
ValGluGluAlaAsnLysGlyGluAsnThrSerLeuLeuHisProValSerLeu
HisGlyMetAspAspProGluArgGluValLeuGluTrpArgPheAspSerArg
LeuAlaPheHisHisValAlaArgGluLeuHisProGluTyrPheLysAsnCys
```

ou un fragment polypeptidique immunogène de cet antigène, caractérisé par:
- la transformation d'un hôte procaryote ou eucaryote avec un vecteur approprié préalablement modifié par une séquence d'ADN s'étendant entre les positions 8324 et 8971 de la séquence de nucléotides définie à la figure 4, ou d'un fragment nucléotidique de cette séquence, dans des conditions permettant la production de l'antigène désiré,
- la récupération et la purification de l'antigène ainsi obtenu par exemple par électrophorèse.

7. Procédé pour la préparation d'un ADN purifié codant pour l'antigène F, ou un fragment de l'antigène F, caractérisé par:
- le clivage de l'ADN entier correspondant au génome complet d'une espèce de LAV par la digestion avec une enzyme de restriction appropriée,
- la récupération des fragments codant pour l'antigène F ou d'un fragment de cet antigène.

8. Procédé selon la revendication 7, caractérisé en ce que l'ADN récupéré a la structure nucléotidique du fragment s'étendant entre les positions 8324 et 8971 de la séquence nucléotidique définie à la figure 4.

9. Procédé pour la préparation d'un vecteur recombinant, caractérisé en ce que le vecteur est modifié par une séquence d'ADN obtenue par un procédé selon la revendication 7 ou la revendication 8.

10. Procédé pour la préparation d'un microorganisme recombinant, caractérisé en ce que le microorganisme est transformé par un vecteur, tel qu'obtenu par le procédé selon la revendication 9 dans des conditions permettant l'expression dans ce microorganime de la séquence d'ADN incluse dans le vecteur et codant pour le dit antigène F ou un fragment immunogène de cet antigène.

11. Procédé selon la revendication 10, caractérisé en ce que les cellules eucaryotes, en particulier les cellules de mammifère ou les levures sont transformées.

12. Procédé pour la détection de la présence d'anticorps anti-LAV dans un milieu biologique, qui comprend la mise en contact du dit milieu biologique avec le polypeptide selon l'une quelconque des revendications 1 à 5 dans des conditions appropriées pour permettre la formation d'un complexe entre les dits anticorps et le dit polypeptide, et la détection du dit complexe comme indication de la présence des dits anticorps dans le dit fluide biologique.

13. Procédé pour la préparation d'une composition de vaccin caractérisée en ce que le principe actif est constitué par l'antigène F ou un fragment immunogène de cet antigène tel qu'obtenu par le procédé selon l'une quelconque des revendications 1 à 5, caractérisé en ce qu'il est en association avec un véhicule pharmaceutiquement acceptable ou physiologiquement acceptable.

14. Procédé pour la production d'anticorps monoclonaux contre l'antigène F tel qu'obtenu par le procédé selon la revendication 1 ou contre un fragment polypeptidique immunogène de cet antigène, comprenant les étapes de:
- fusion de cellules de myélome avec des splénocytes d'un animal préalablement immunisé avec l'antigène F, pour obtenir des hybridomes,
- sélection des hybridomes produisant les anticorps ayant la spécificité pour l'antigène F, ou d'un fragment polypeptidique immunogène de cet antigène,
- récupération des anticorps reconnus par l'antigène F ou par un fragment polypeptidique immunogène de cet antigène.

15. Utilisation d'anticorps selon les revendications 12 ou 14, pour l'identification de l'antigène F ou même pour la détermination de la proportion relative de cet antigène, dans un échantillon biologique contenant LAV ou des virus apparentés.

FIG.1.

EP 0 387 915 B1

FIG.2.

H = Hind III    K = Kpn I
S = Sac I       R = EcoRI
B = Bam HI      Sa = Sal I
P = Pst I       X = Xho I
Bg = Bgl II

# FIG. 3.

EP 0 387 915 B1

EP 0 387 915 B1

HindIII

CGTCTCTCTCCGTTAGACCACATTTGAGCCTGCCAGCTCTCTGGCTAACTAGGGAACCCACTGCTTAAGCCTCAATAAAGCTTGCCTTGA...GCTTCAAGTAGTGTGTGCCCCGTCTGTTGT
100

GTCACTCTCGTAACTAGACATCCCTCAGACCCTTTTAGTCAGTGTGGAAAATCTCTAGCAGTGGCCGCCCGAACAGCGACTTCAAAGCGAAAGGCAAACCAGAGGACCTCTCTCGACGCAG
200

GAG ► LeuAlaGluAlaArgArgArgGluMetGlyAlaArgAlaSerValLeuSer
GACTCGCCTTGCTGAAGCCGCGCACCGCAAGAGCCGAGCGGGAGCCCACTGCTCACTACGCCAAAAATTTTGACTAGCCGGAGGCTAGAACGAGAGAGATCGCGTGCCGACAGCCGTCACTATTAA
300

GlyGlyGluLeuAspArgTrpGluLysIleArgLeuArgProGlyGlyLysLysTyrLysLeuLysHisIleValTrpAlaSerArgGluLeuGluArgPheAlaValAsnProGly
CCCGCGGCACAATTAGATCCATGGGAAAAAATTCGGTTAAGGCCAGCGGGCAAACAAAAATATAAATTAAAACATATAGTATGGGCAAGCAGCGAGCTAGAACGATTCGCACTTAATCCTG
400

LeuLeuGluThrSerGluGlyCysArgGlnIleLeuGlyGlnLeuGlnProSerLeuGlnThrGlySerGluGluLeuArgSerLeuTyrAsnThrValAlaThrLeuTyrCysValHis
GCCTGTTAGAAACATCACAAGGCTGTAGACAAATACTGGGACAGCTACAACCATCCCTTCAGACACGGATCAGAAGAACTTAGATCATTATATAATACAGTAGCAACCCTCTATTGTGTGCC
500                                                                                                    600

GlnArgIleGluIleLysAspThrLysGluAlaLeuAspLysIleGluGluGluGlnAsnLysSerLysLysLysAlaGlnGlnAlaAlaAlaAspThrGlyHisSerSerGlnValSer
ATCAAACGATAGAGATAAAAGACACCAAGGAAGCTTTAGACAAGATAGAGGAAGAGCAAAACAAAAGTAAGAAAAAAGCACAGCAAGCAGCAGCTGACACACGGACACAGCCACCCAGGTCA
700

GlnAsnTyrProIleValGlnAsnIleGlnGlyGlnMetValHisGlnAlaIleSerProArgThrLeuAsnAlaTrpValLysValValGluGluLysAlaPheSerProGluValIle
GCCAAAATTACCCTATAGTGCAGAACATCCAGGGGCAAATGGTACATCAGGCCATATCACCTAGAACTTTAAATGCATGGGTAAAAGTAGTAGAAGAGAAGGCTTTCAGCCCAGAAGTGA
800

ProMetPheSerAlaLeuSerGluGlyAlaThrProGlnAspLeuAsnThrMetLeuAsnThrValGlyGlyHisGlnAlaAlaMetGlnMetLeuLysGluThrIleAsnGluGluAla
TACCCATGTTTTCAGCATTATCAGAAGGAGCCACCCCACAAGATTTAAACACCATGCTAAACACAGTGGGGGGACATCAAGCAGCCATGCAAATGTTAAAAGAGACCATCAATGAGGAAG
900

AlaGluTrpAspArgValHisProValHisAlaGlyProIleAlaProGlyGlnMetArgGluProArgGlySerAspIleAlaGlyThrThrSerThrLeuGlnGluGlnIleGlyTrp
CTGCAGAATGGGATAGAGTGCATCCAGTGCATGCAGGGCCTATTGCACCAGGCCAGATGAGAGAACCAAGGGGAAGTGACATAGCAGGAACTACTAGTACCCTTCACCAACAAATAGGAT
1000

MetThrAsnAsnProProIleProValGlyGluIleTyrLysArgTrpIleIleLeuGlyLeuAsnLysIleValArgMetTyrSerProThrSerIleLeuAspIleArgGlnGlyPro
GGATGACAAATAATCCACCTATCCCAGTAGGAGAAATTTATAAAAGATGGATAATCCTGGGATTAAATAAAATAGTAAGAATGTATAGCCCTACCAGCATTCTGGACATAAGACAAGGAC
1100                                                                                                 1200

LysGluProPheArgAspTyrValAspArgPheTyrLysThrLeuArgAlaGluGlnAlaSerGlnGluValLysAsnTrpMetThrGluThrLeuLeuValGlnAsnAlaAsnProAsp
CAAAAGAACCCTTTAGAGACTATGTAGACCGGTTCTATAAAACTCTAAGAGCCGAGCAAGCTTCACAGGAGGTAAAAAATTGGATGACAGAAACCTTGTTGGTCCAAAATGCGAACCCAG
1300

CysLysThrIleLeuLysAlaLeuGlyProAlaAlaThrLeuGluGluMetMetThrAlaCysGlnGlyValGlyGlyProGlyHisLysAlaArgValLeuAlaGluAlaMetSerGln
ATTGTAAGACTATTTTAAAAGCATTGGGACCAGCAGCTACACTAGAAGAAATGATGACAGCATGTCAGGGAGTGGGAGGACCCGGCCATAAGGCAAGAGTTTTGGCTGAAGCAATGAGCC
1400

ValThrAsnSerAlaThrIleMetMetGlnArgGlyAsnPheArgAsnGlnArgLysIleValLysCysPheAsnCysGlyLysGluGlyHisIleAlaArgAsnCysArgAlaProArg
AAGTAACAAATTCAGCTACCATAATGATGCAAAGAGGCAATTTTAGGAACCAAAGAAAGATTGTTAAGTGTTTCAATTGTGGCAAAGAACGGCACATAGCCAGAAATTGCAGGGCCCCTA
1500

POL ► PhePheArgGluAspLeuAlaPheLeuGlnGlyLysAlaArgGluPheSer
LysLysGlyCysTrpLysCysGlyLysGluGlyHisGlnMetLysAspCysThrGluArgGlnAlaAsnPheLeuGlyLysIleTrpProSerTyrLysGlyArgProGlyAsnPheLeu
CGAAAAAGGGCTGTTGGAAATGTGGAAAGGAAGGACACCAAATGAAAGATTGTACTGAGAGACAGGCTAATTTTTTAGGGAACATCTGCCCTTCCTACAAGGGAAGGCCAGGGAATTTTC
1600

SerGluGlnThrArgAlaAsnSerProThrArgArgGluLeuGlnValTrpGlyArgAspAsnAsnSerLeuSerGluAlaGlyAlaAspArgGlnGlyThrValSerPheAsnPhePro
GlnSerArgProGluProThrAlaProProGluGluSerPheArgSerGlyValGluThrThrThrProSerGlnLysGlnGluProIleAspLysGluLeuTyrProLeuThrSerLeu
TTCAGAGCCAGACCAGAGCCAACAGCCCCACCAGAAGAGAGCTTCAGGTCTGGGGTACAGACAACAACTCCCTCTCAGAAGCACCAGCCCGATAGACAAGCAACTGTATCCTTTAACTTCCC
1700                                                                                                1800

GlnIleThrLeuTrpGlnArgProLeuValThrIleLysIleGlyGlyGlnLeuLysGluAlaLeuLeuAspThrGlyAlaAspAspThrValLeuGluGluMetSerLeuProGlyArg
ArgSerLeuPheGlyAsnAspProSerSerGln ●
TCAGATCACTCTTTCGGCAACGACCCCTCGTCACAATAAAGATAGGGGGGCAACTAAAGGAAGCTCTATTAGATACAGGAGCAGATGATACACTATTAGAAGAAATGAGTTTGCCAGGAAG
1900

FIG. 4a

FIG. 1 b

TrpLysProLysMetIleGlyGlyIleGlyGlyPheIleLysValArgGlnTyrAspGlnIleLeuIleGluIleCysGlyHisLysAlaIleGlyThrValLeuValGlyProThrPro
ATGGAAACCAAAAATGATAGGCGGAATTGGAGGTTTTATCAAAGTAAGACAGTATGATCAGATACTCATAGAAATCTGTGGACATAAAGCTATAGGTACAGTATTAGTAGGACCTACACC
2000

ValAsnIleIleGlyArgAsnLeuLeuThrGlnIleGlyCysThrLeuAsnPheProIleSerProIleGluThrValProValLysLeuLysProGlyMetAspGlyProLysValLys
TGTCAACATAATTGGAAGAAATCTGTTGACTCAGATTGGTTGCACTTTAAATTTTCCCATTAGTCCTATTGAAACTGTACCAGTAAAATTAAAGCCAGGAATGGATGGCCCAAAAGTTAA
2100

GlnTrpProLeuThrGluGluLysIleLysAlaLeuValGluIleCysThrGluMetGluLysGluGlyLysIleSerLysIleGlyProGluAsnProTyrAsnThrProValPheAla
ACAATGCCCATTGACACAAGAAAAAATAAAAGCATTAGTAGAAATTTGTACAGAAATGGAAAAGGAAGGGAAAATTTCAAAAATTGGGCCTGAAAATCCATACAATACTCCAGTATTTGC
2200

IleLysLysLysAspSerThrLysTrpArgLysLeuValAspPheArgGluLeuAsnLysArgThrGlnAspPheTrpGluValGlnLeuGlyIleProHisProAlaGlyLeuLysLys
CATAAAGAAAAAAGACAGTACTAAATGGAGAAAATTAGTAGATTTCAGAGAACTTAATAAGAGAACTCAAGACTTCTGGGAAGTTCAATTAGGAATACCACATCCCGCAGGGTTAAAAAA
2300                                                                                                                    2400

LysLysSerValThrValLeuAspValGlyAspAlaTyrPheSerValProLeuAspGluAspPheArgLysTyrThrAlaPheThrIleProSerIleAsnAsnGluThrProGlyIle
GAAAAAATCAGTAACAGTACTGGATGTGGGTGATGCATATTTTTCAGTTCCCTTAGATGAAGACTTCAGGAAGTATACTGCATTTACCATACCTAGTATAAACAATGAGACACCAGGCAT
2500

ArgTyrGlnTyrAsnValLeuProGlnGlyTrpLysGlySerProAlaIlePheGlnSerSerMetThrLysIleLeuGluProPheArgLysGlnAsnProAspIleValIleTyrGln
TACATATCAGTACAATGTGCTTCCACAGGGATGGAAAGGATCACCAGCAATATTCCAAAGTAGCATGACAAAAATCTTAGAGCCCTTTTAGAAAACAAAATCCAGACATAGTTATCTATCA
2600

TyrMetAspAspLeuTyrValGlySerAspLeuGluIleGlyGlnHisArgThrLysIleGluGluLeuArgGlnHisLeuLeuArgTrpGlyLeuThrThrProAspLysLysHisGln
ATACATGGATGATTTGTATGTAGGATCTGACTTAGAAATACGGCAGCATAGAACAAAAATAGAGGAGCTGAGACAACATCTCTTGAGGTCGGGGACTTACCACACCAGACAAAAAACATCA
2700

LysGluProProPheLeuTrpMetGlyTyrGluLeuHisProAspLysTrpThrValGlnProIleValLeuProGluLysAspSerTrpThrValAsnAspIleGlnLysLeuValGly
GAAAGAACCTCCATTCCTTTGGATGGGTTATGAACTCCATCCTGATAAATGGACAGTACAGCCTATAGTGCTGCCAGAAAAAGACAGCTGGACTGTCAATCACATACACAAGTTAGTGGG
2800

LysLeuAsnTrpAlaSerGlnIleTyrProGlyIleLysValArgGlnLeuCysLysLeuLeuArgGlyThrLysAlaLeuThrGluValIleProLeuThrGluGluAlaGluLeuGlu
AAAATTGAATTGGGCAAGTCAGATTTACCCAGGGATTAAAGTAAGGCAATTATGTAAACTCCTTAGAGGAACCAAAGCACTAACAGAAGTAATACCACTAACAGAAGAACCAGAGCTAGA
2900                                                                                                                    3000

LeuAlaGluAsnArgGluIleLeuLysGluProValHisGlyValTyrTyrAspProSerLysAspLeuIleAlaGluIleGlnLysGlnGlyGlnGlyGlnTrpThrTyrGlnIleTyr
ACTGGCAGAAAACAGAGAGATTCTAAAAGAACCAGTACATGGAGTGTATTATGACCCATCAAAAGACTTAATAGCAGAAATACAGAAGCAGGGGCAACGCCAATCGACATATCAAATTTA
3100

GlnGluProPheLysAsnLeuLysThrGlyLysTyrAlaArgThrArgGlyAlaHisThrAsnAspValLysGlnLeuThrGluAlaValGlnLysIleThrThrGluSerIleValIle
TCAAGAGCCATTTAAAAATCTGAAAACAGGGAAAATATGCAAGAACGAGGGGTGCCCACACTAATGATGTAAAACAATTAACAGAGGCAGTGCAAAAAATAACCACAGAAAGCATAGTAAT
3200

TrpGlyLysThrProLysPheLysLeuProIleGlnLysGluThrTrpGluThrTrpTrpThrGluTyrTrpGlnAlaThrTrpIleProGluTrpGluPheValAsnThrProProLeu
ATGGGGAAAGACTCCTAAATTTAAACTACCCATACAAAAGGAAACATGGGAAACATGGTGGACAGAGTATTGGCAAGCCACCTGGATTCCTGAGTGGGAGTTTGTCAATACCCCTCCTTT
3300

ValLysLeuTrpTyrGlnLeuGluLysGluProIleValGlyAlaGluThrPheTyrValAspGlyAlaAlaSerArgGluThrLysLeuGlyLysAlaGlyTyrValThrAsnArgGly
AGTGAAATTATGGTACCAGTTAGAGAAAGAACCCATACTAGGAGCAGAAACGTTCTATGTAGATGGGGCAGCTAGCAGGGAGACTAAATTAGGAAAAGCAGGATATGTTACTAATAGAGG
3400

ArgGlnLysValValThrLeuThrAspThrThrAsnGlnLysThrGluLeuGlnAlaIleHisLeuAlaLeuGlnAspSerGlyLeuGluValAsnIleValThrAspSerGlnTyrAla
AAGACAAAAAGTTGTCACCCTAACTGACACAACAAATCAGAAGACTGAGTTACAAGCAATTCATCTAGCTTTGCAGGATTCGGGATTAGAAGTAAATATAGTAACAGACTCACAATATGC
3500                                                                                                                    3600

LeuGlyIleIleGlnAlaGlnProAspLysSerGluSerGlu   ValAsnGlnIleIleGluGlnLeuIleLysLysGluLysV-'TyrLeuAlaTrpValProAlaHisLysGlyIle
ATTAGGAATCATTCAACCACAACCAGATAAAAGTGAATCAGAGTAAGTCAATCAAATAATAGAGCCAGTTAATAAAAAAGGAAAAGC  TATCTCGCCATCGGTACCAGCACACAAAGGAAT
                                                                                        3700

GlyGlyAsnGluGlnValAspLysLeuValSerAlaGlyIleArgLysValLeuPheLeuAspGlyIleAspLysAlaGlnAspGluHisGluLysTyrHisSerAsnTrpArgAlaMet
TCGCAGGAAATGAACAAGTAGATAAATTAGTCACTGGTCGAATCAGGAAAGTACTATTTTTAGATGGAATAGATAAGCCCCAAGATGAACATGAGAAATATCACACGTAATTCGAGAGCAAT
                                                                                        3800

AlaSerAspPheAsnLeuProProValValAlaLysGluIleValAlaSerCysAspLysCysGlnLeuLysGlyGluAlaMetHisGlyGlnValAspCysSerProGlyIleTrpGln
CGCTAGTCGATTTTAACCTGCCACCTGTAGTAGCAAAAGAAATACTAGCCAGCTGTCATAAATGTCACGCTAAAAGGAGAAGCCATCCATGGACAAGTAGACTGTAGTCCACGAATATGGCA
                                                                                        3900

LeuAspCysThrHisLeuGluGlyLysValIleLeuValAlaValHisValAlaSerGlyTyrIleGluAlaGluValIleProAlaGluThrGlyGlnGluThrAlaTyrPheLeuLeu
ACTAGATTGTACACATTTAGAAGGAAAAGTTATCCTGCTAGCAGTTCATGTAGCCAGTCGGATATATAGAAGCAGAAGTTATTCCAGCAGAAACAGGGCAGGAAACAGCCATACTTTCTTTT
                                                                                        4000

LysLeuAlaGlyArgTrpProValLysThrIleHisThrAspAsnGlySerAsnPheThrSerThrThrValLysAlaAlaCysTrpTrpAlaGlyIleLysGlnGluPheGlyIlePro
AAAATTAGCAGGAAGATGGCCAGTAAAAACAATACATACAGACAATGGCAGCAATTTCACCAGTACTACGGTTAACGCCGCCTGTTGCGTGGGCGGGAATCAAGCAGGAATTTGGAATTCC
                                                                                        4200

TyrAsnProGlnSerGlnGlyValValGluSerMetAsnLysGluLeuLysLysIleIleGlyGlnValArgAspGlnAlaGluHisLeuLysThrAlaValGlnMetAlaValPheIle
CTACAATCCCCAAAGTCAAGGAGTAGTAGAATCTATGAATAAAGAATTAAAGAAAATTTATAGGCCAGCTAAGAGATCAGCCTGAACATCTTAAGACAGCAGTACAAATGGCAGTATTCAT
                                                                                        4300

HisAsnPheLysArgLysGlyGlyIleGlyGlyTyrSerAlaGlyGluArgIleValAspIleIleAlaThrAspIleGlnThrLysGluLeuGlnLysGlnIleThrLysIleGlnAsn
CCACAATTTTAAAAGAAAAGGCGGGATTCGGGGGGTACAGTGCACGGGAAAGAATAGTAGACATAATAGCCAACAGACATACAAACTAAAGAATTACAAAAACAAATTACAAAAATTCAAAA
                                                                                        4400

PheArgValTyrTyrArgAspSerArgAspProLeuTrpLysGlyProAlaLysLeuLeuTrpLysGlyGluGlyAlaValValIleGlnAspAsnSerAspIleLysValValProArg
                                                                                                      ORF Q— CysGlnGlu

TTTTCGGGTTTATTACAGCGACAGCAGAGATCCACTTTGGAAAGGACCAGCAAAGCTCCTCTGGAAAGGTGAAGGGGCAGTAGTAATACAAGATAATAGTGACATAAAAGTAGTGCCAAAG
                                                                                        4500

ArgLysAlaLysIleIleArgAspTyrGlyLysGlnMetAlaGlyAspAspCysValAlaSerArgGlnAspGluAsp •
GluLysGlnArgSerLeuGlyIleGluMetGluAsnArgTrpGlnValMetIleValTrpGlnValAspArgMetArgIleArgThrTrpLysSerLeuValLysHisHisMetTyrValSer
AAGAAAAGCAAAGATCATTAGGGATTATGGAAAACAGATGGCAGGTGATGATTGTGTGGCAAGTAGACAGGATGAGGATTAGAACATGGAAAACTTTAGTAAAACACCATATGTATGTTT
                                                                                        4600

GlyLysAlaArgGlyTrpPheTyrArgHisHisTyrGluSerProHisProArgIleSerSerGluValHisIleProLeuGlyAspAlaArgLeuValIleThrThrTyrTrpGlyLeu
CACCGAAAGCTAGGGGATCGTTTTATAGACATCACTATGAAAGCCCTCATCCAAGAATAAGTTCAGAAGTACACATCCCACTAGGGGATGCTAGATTGGTAATAACAACATATTGGGGTC
                                                                                        4800

HisThrGlyGluArgAspTrpHisLeuGlyGlnGlyValSerIleGluTrpArgLysLysArgTyrSerThrGlnValAspProGluLeuAlaAspGlnLeuIleHisLeuTyrTyrPhe
TCCATACAGGAGAAAGACACTGGCATCTCGGTCAGGGAGTCTCCATAGAATGGAGGAAAAAGAGATATAGCACACAAGTAGACCCTGAACTAGCAGACCAACTAATTCATCTGTATTACT
                                                                                        4900

AspCysPheSerAspSerAlaIleArgLysAlaLeuLeuGlyHisIleValSerProArgCysGluTyrGlnAlaGlyHisAsnLysValGlySerLeuGlnTyrLeuAlaLeuAlaAla
TTGACTGTTTTTCAGACTCTGCTATAAGAAAGCCCTTATTAGGACATATAGTTAGCCCTAGGTGTGAATATCAAGCAGGACATAACAAGGTAGGATCTCTACAATACTTGGCACTAGCAG
                                                                                        5000

LeuIleThrProLysLysIleLysProProLeuProSerValThrLysLeuThrGluAspArgTrpAsnLysProGlnLysThrLysGlyHisArgGlySerHisThrMetAsnGlyHis
CATTAATAACACCAAAAAAGATAAAGCCACCTTTGCCTAGTGTTACGAAACTGACACGGGATAGATGGAACAAGCCCCAGAAGACCAAGCGCCACAGAGGCAGCCACACAATGAATGGCAC
                                                                                        5100

ACTAGAGCTTTTAGACGGAGCTTAAGAATGAAGCTGTTACACATTTTCCTAGGATTTGGCTCCATGCCTTAGCGCCAACATATCTATGAAACTTATCGGGATACTTGCGCCAGCAGTGGCAAGC
                                                                                        5200

CATAATAAGAATTCTGCAACAACTGCTGTTTATCCATTTCAGAATTGGGTGTCGACATAGCAGAATAGGCGTTACTCAACAGAGGAGAGCAAGAAATGGAGCCAGTAGATCCTAGACTAG
                                                                                        5400

AGCCCTGGAACCATCCAGGAAGTCAGCCTAAAACTGCTTGTACCACTTCCTATTGTAAAAAGTGTTGCTTTCATTGCCAAGTTTGTTTCACAACAAAAGCCTTAGCCATCTCCTATGCCA
                                                                                        5500

GGAAGAAGCCCGAGACACCGACGAAGACCTCCTCAAGGCAGTCAGACTCATCAAGTTTCTCTATCAAAGCAGTAAGTACTACATGTAATGCAACCTATACAAATAGCAATAGCAGCATTAC
                                                                                        5600

FIG. 4 c

EP 0 387 915 B1

ENV ► LysGluGlnLysThr
TAGTAGCAATAATAATAGCCAATAGTTGTCTCGTCCATAGTAATCATAGAATATAGGAAAATATTAAGACAAAGAAAAATAGACACGTTAATTCATACACTAATAGAAAGAGCCACAAGACA
                                      5700
ValAlaMetArgValLysGluLysTyrGlnHisLeuLeuTrpArgTrpGlyTrpLysTrpGlyThrMetLeuLeuGlyIleLeuMetIleCysSerAlaThrGluLysLeuTrpValThrVal
GTCCCAATGACAGTCAACGACAAATATCACCACTTGTGGAGATGGCGGGTGGAAATGGGGCACCATGCTCGTTGGGATATTGATCATCTGTAGTCCTACAGAAAAAATTGTCGGGTCACAGTC
                                      5800
TyrTyrGlyValProValTrpLysGluAlaThrThrThrLeuPheCysAlaSerAspAlaLysAlaTyrAspThrGluValHisAsnValTrpAlaThrHisAlaCysValProThrAsp
TATTATGGGGTACCTGTCTGGAAGGAAGCCAACCACCACTCTATTTTGTGCATCAGATGCTAAAGCATATCATACAGAGGTACATAATGTTTGGGCCACACATGCCTGTGTACCCACAGAC
        5900                                                                                    6000
ProAsnProGlnGluValValLeuValAsnValThrGluAsnPheAsnMetTrpLysAsnAspMetValGluGlnMetHisGluAspIleIleSerLeuTrpAspGlnSerLeuLysPro
CCCAACCCACAAGAAGTAGTATTGGTAAATGTCACAGAAAATTTTAACATGTGGAAAAATGACATGGTAGAACAGATGCATGAGGATATAATCACTTTATGGGATCAAAGCCTAAAGCCA
                                                                      6100
CysValLysLeuThrProLeuCysValSerLeuLysCysThrAspLeuGlyAsnAlaThrAsnThrAsnSerSerAsnThrAsnSerSerSerGlyGluMetMetMetGluLysGlyGlu
TGTGTAAAATTAACCCCACTCTGTGTGTTAGTTTAAAGTGCACTGATTTGGGGAATGCTACTAATACCAATAGTAGTAATACCAATAGTAGTAGCGGGCGAAATGATCATGGAGAAAGGAGAG
                                                        6200
IleLysAsnCysSerPheAsnIleSerThrSerIleArgGlyLysValGlnLysGluTyrAlaPhePheTyrLysLeuAspIleIleProIleAspAsnAspThrThrSerTyrThrLeu
ATAAAAAACTGCTCTTTCAATATCAGCACAAGCATAAGAGGTAAGGTGCAGAAAGAATATGCCATTTTTTTATAAACTTGATATAATACCAATAGATAATGATACTACCAGCTATACGTTG
                        6300
ThrSerCysAsnThrSerValIleThrGlnAlaCysProLysValSerPheGluProIleProIleHisTyrCysAlaProAlaGlyPheAlaIleLeuLysCysAsnAsnLysThrPhe
ACAAGTTGTAACACCTCAGTCATTACACAGGCCTGTCCAAAGGTATCGTTTGAGCCAATTCCCATACATTATTGTGCCCCGGCTGGTTTTGCGATTCTAAAATGTAATAATAAGACGTTC
                  6400
AsnGlyThrGlyProCysThrAsnValSerThrValGlnCysThrHisGlyIleArgProValValSerThrGlnLeuLeuLeuAsnGlySerLeuAlaGluGluGluValValIleArg
AATGGAACAGGACCATGTACAAATGTCAGCACAGTACAATGTACACATGGAATTAGGCCAGTAGTATCAACTCAACTGCTGTTGAATGGCAGTCTAGCAGAAGAAGAGGTAGTAATTAGA
                              6500                                                          6600
SerAlaAsnPheThrAspAsnAlaLysThrIleIleValGlnLeuAsnGlnSerValGluIleAsnCysThrArgProAsnAsnAsnThrArgLysSerIleArgIleGlnArgGlyPro
TCTGCCAATTTCACAGACAATGCTAAAACCATAATAGTACAGCTGAACCAATCTGTAGAAATTAATTGTACAAGACCCAACAACAATACAAGAAAAGTATCCGTATCCACAGCGGGACCA
                                              6700
GlyArgAlaPheValThrIleGlyLysIleGlyAsnMetArgGlnAlaHisCysAsnIleSerArgAlaLysTrpAsnAlaThrLeuLysGlnIleAlaSerLysLeuArgGluGlnPhe
GGGACAGCCATTTGTTACAATAGGAAAAATAGGAAATATGAGACAAGCACATTGTAACATTAGTACAGCAAAATGGAATGCCACTTTAAAACAGATAGCTAGCAAATTAAGAGAACAATTT
                                                  6800
GlyAsnAsnLysThrIleIlePheLysGlnSerSerGlyGlyAspProGluIleValThrHisSerPheAsnCysGlyGlyGluPhePheTyrCysAsnSerThrGlnLeuPheAsnSer
GGAAATAATAAAACAATAATCTTTAAGCAATCCTCAGGAGGGGACCCAGAAATTGTAACGCACAGTTTTAATTGTGGAGGGGAATTTTTCTACTGTAATTCAACACAACTGTTTAATAGT
                                  6900
ThrTrpPheAsnSerThrTrpSerThrGluGlySerAsnAsnThrGluGlySerAspThrIleThrLeuProCysArgIleLysGlnPheIleAsnMetTrpGlnGluValGlyLysAla
ACTTGGTTTAATAGTACTTGGAGTACTGAAGGGTCAAATAACACTGAAGGAAGTGACACAATCACACTCCCATGCAGAATAAAACAATTTATAAACATGTGGCAGGAAGTAGGAAAAGCA
                  7000
MetTyrAlaProProIleSerGlyGlnIleArgCysSerSerAsnIleThrGlyLeuLeuLeuThrArgAspGlyGlyAsnAsnAsnAsnGlySerGluIlePheArgProGlyGlyGly
ATGTATGCCCCTCCCATCAGCGGACAAATTAGATGTTCATCAAATATTACAGGGCTGCTATTAACAAGAGATGGTGGTAATAACAACAATGGGTCCGAGATCTTCAGACCTGGAGGAGGA
          7100                                                                            7200
AspMetArgAspAsnTrpArgSerGluLeuTyrLysTyrLysValValLysIleGluProLeuGlyValAlaProThrLysAlaLysArgArgValValGlnArgGluLysArgAlaVal
GATATGAGGGACAATTGGAGAAGTGAATTATATAAATATAAAGTAGTAAAAATTGAACCATTAGGAGTAGCACCCACCAAGGCAAAGACAAGACTGGTCCAGAGAGAAAAAAGAGCAGTG
                                                              7300

FIG. 4 d

EP 0 387 915 B1

GlyIleGlyAlaLeuPheLeuGlyPheLeuGlyAlaAlaGlySerThrMetGlyAlaArgSerMetThrLeuThrValGlnAlaArgGlnLeuLeuSerGlyIleValGlnGlnGlnAsn
CGAATACCAGCTTTCTTCCTTCGGTTCTTCGGAGCAGCAGGAAGCACTATCGGCGCACCGTCAATCACGCTGACGGTACAGCCCAGACAATTATTCTCTGGTATAGTCCACCAGCAGAAC
                        7400
AsnLeuLeuArgAlaIleGluAlaGlnGlnHisLeuLeuGlnLeuThrValTrpGlyIleLysGlnLeuGlnAlaArgIleLeuAlaValGluArgTyrLeuLysAspGlnGlnLeuLeu
AATTTGCTGAGCGGCTATTGAGCGCCCAACACCATCTGTTGCAACTCACAGTCTGGGGCATCAAGCAGCTCCAGGCAAGAATCCTGGCTGTGGAAAGATACCTAAAGGATCAACAGCTCCTG
                        7500
GlyIleTrpGlyCysSerGlyLysLeuIleCysThrThrAlaValProTrpAsnAlaSerTrpSerAsnLysSerLeuGluGlnIleTrpAsnAsnMetThrTrpMetGluTrpAspArg
GGCATTTGGCGGTTGCTCTGGAAAAGTCATTTGCACCACTGCTGTGCCTTGGAATGCTAGTTCGAGTAATAAATCTCTGCAACAGATTTGGAATAACATGACCTGGATGGAGTGGGACACA
                        7600
GluIleAsnAsnTyrThrSerLeuIleHisSerLeuIleGluGluSerGlnAsnGlnGlnGluLysAsnGluGlnGluLeuLeuGluLeuAspLysTrpAlaSerLeuTrpAsnTrpPhe
GAAATTAACAATTACACAAGCTTAATACATTCGTTAATTGAAGAATCGCAAAACCAGCAAGAAAAGAATGAACAAGAATTATTGGAATTAGATAAATGGGCCAAGTTTGTGGAATTGGTTT
                        7700                                                                                    7800
AsnIleThrAsnTrpLeuTrpTyrIleLysIlePheIleMetIleValGlyGlyLeuValGlyLeuArgIleValPheAlaValLeuSerIleValAsnArgValArgGlnGlyTyrSer
AACATAACAAATTGGCTGTGGTATATAAAAATATTCATAATGATAGTAGGAGGCTTCGTAGGTTTAAGAATAGTTTTTGCTGTACTTTCTATAGTGAATAGAGTTAGCGAGCGGATATTCA
                                                                                        7900
ProLeuSerPheGlnThrHisLeuProThrProArgGlyProAspArgProGluGlyIleGluGluGluGlyGlyGluArgAspArgAspArgSerIleArgLeuValAsnGlySerLeu
CCATTATCGTTTCAGACCCACCTCCCAACCCCGACGCGACCCGACAGGCCCGAAGGAATAGAAGAAGAAGGTGGAGAGAGAGACAGAGACAGATCCATTCGATTAGTCAACGGCATCCTTA
                                                                        8000
AlaLeuIleTrpAspAspLeuArgSerLeuCysLeuPheSerTyrHisArgLeuArgAspLeuLeuLeuIleValThrArgIleValGluLeuLeuGlyArgArgGlyTrpGluAlaLeu
GCACTTATCTGGGACGATCTGCGGAGCCTGTGCCTCTTCAGCTACCACCGCTTGAGAGACTTACTCTTGATTGTAACGAGGATTGTCGAACTTCTGGGACGCAGGGCGTCGCAAGCCCTC
                                                                8100
LysTyrTrpTrpAsnLeuLeuGlnTyrTrpSerGlnGluLeuLysAsnSerAlaValSerLeuLeuAsnAlaThrAlaIleAlaValAlaGluGlyThrAspArgValIleGluValVal
AAATATTGGTGGAATCTCCTACAGTATTGGAGTCAGGAACTAAAGAATAGTGCTGTTAGCTTGCTCAATGCCACAGCCATAGCAGTAGCTGAGGCGACAGATAGGGTTATAGAAGTAGTA
                                                        8200
GlnGlyAlaCysArgAlaIleArgHisIleProArgArgIleArgGlnGlyLeuGluArgIleLeuLeu •
                ORF F ▸ AspArgAlaTrpLysGlyPheCysTyrLys[Met]GlyGlyLysTrpSerLysSerSerValValGlyTrpProThrVal
CAAGGAGCTTGTAGAGCTATTCGCCACATACCTAGAAGAATAAGACAGGGCTTGGAAAGGATTTTGCTATAAGATGGGTGGCAAGTGGTCAAAAAGTAGTCTCGTTGGATGGCCTACTGT
                8300                                                                            8400
ArgGluArgMetArgArgAlaGluProAlaAlaAspGlyValGlyAlaAlaSerArgAspLeuGluLysHisGlyAlaIleThrSerSerAsnThrAlaAlaThrAsnAlaAlaCysAla
AAGCGGAAAGAATGAGACCGAGCTGAGCCAGCAGCAGATGGCGTGCGGAGCACCATCTCGAGACCTGGAAAAACATGGAGCAATCACAAGTAGCAATACAGCAGCTACCAATGCTGCTTGTGCC
                                                                        8500
TrpLeuGluAlaGlnGluGluGluGluValGlyPheProValThrProGlnValProLeuArgProMetThrTyrLysAlaAlaValAspLeuSerHisPheLeuLysGluLysGlyGly
CTGGCTAGAAGCACAAGAGGAGGAGGAGGTGCGGTTTTCCAGTCACACCTCACGTACCTTTAAGACCAATGACTTACAAGCGCAGCTGTAGATCTTAGCCACTTTTTTAAAAGAAAAGGGGG
                                                                8600
LeuGluGlyLeuIleHisSerGlnArgArgGlnAspIleLeuAspLeuTrpIleTyrHisThrGlnGlyTyrPheProAspTrpGlnAsnTyrThrProGlyProGlyValArgTyrPro
ACTGGAAGGGCTAATTCACTCCCAACCAAGACAAGATATCCTTGATCTGTGGATCTACCACACACAAGGCTACTTCCCTGATTGGCAGAACTACACACCAGGGCCAGGGGTCAGATATCC
                                                        8700
LeuThrPheGlyTrpCysTyrLysLeuValProValGluProAspLysValGluGluAlaAsnLysGlyGluAsnThrSerLeuLeuHisProValSerLeuHisGlyMetAspAspPro
ACTGACCTTTGGATGGTGCTACAAGCTAGTACCAGTTGAGCCACATAACGTAGAAGAGCCCAATAAAGGAGAGAACACCAGCTTGTTACACCCTGTGAGCCTGCATCGAATGGATGACCCC
                                                8800
GluArgGluValLeuGluTrpArgPheAspSerArgLeuAlaPheHisHisValAlaArgGluLeuHisProGluTyrPheLysAsnCys •
TGAGACAGAAGTGTTAGAGTGGAGGTTTGACAGCCGCCTAGCATTTCATCACGTGCCCCGAGAGCTGCATCCGGAGTACTTCAAGAACTCCTGACATCGAGCTTGCTACAAGGGACTTTC
                8900                                                                            9000
CGCTCGGGACTTTCCACGGGAGGCGTGCCCTGGGCGGGACTCGGGAGTCGCGAGCCCTCAGATGCTGCCATATAAGCAGCTGCTTTTTGCCTGTACTGGGTCTCTCTGGTTAGACCAGATTT
                                                    HindIII •
GAGCCTGGGAGCTCTCTGGCTAACTAGGGAACCCACTGCTT⁀·GCCTCAATAAAGCTTGCCTTGAGTGCTTCA
                        9193

FIG. 5

FIG. 6